(19) Europäisches Patentamt
European Patent Office
Office européen des brevets

(11) EP 1 719 511 B1

(12) **EUROPEAN PATENT SPECIFICATION**

(45) Date of publication and mention of the grant of the patent:
**10.12.2008 Bulletin 2008/50**

(51) Int Cl.:
***A61K 31/4745*** *(2006.01)* ***A61P 31/12*** *(2006.01)*
***A61P 35/00*** *(2006.01)*

(21) Application number: **06010882.6**

(22) Date of filing: **14.11.2002**

(54) **N-[4-(4-amino-2-ethyl-1H-imidazo[4,5-c]quinolin-1-yl)butyl]methanesulfonamide, a pharmaceutical composition comprising the same and use thereof**

N-[4-(4-Amino-2-ethyl-1H-imidazo[4,5-c]chinolin-1-yl)butyl]methanesulfonamide, diese enthaltende pharmazeutische Zusammensetzung und deren Verwendung

N-[4-(4-amino-2-éthyl-1H-imidazo[4,5-c]quinolin-1-yl)butyl]methanesulfonamide, composition pharmaceutique le contenant et son utilisation

(84) Designated Contracting States:
**AT BE BG CH CY CZ DE DK EE ES FI FR GB GR IE IT LI LU MC NL PT SE SK TR**

(30) Priority: **16.11.2001 US 332412 P**

(43) Date of publication of application:
**08.11.2006 Bulletin 2006/45**

(62) Document number(s) of the earlier application(s) in accordance with Art. 76 EPC:
**02780689.2 / 1 455 700**

(73) Proprietor: **3M Innovative Properties Company**
**St. Paul MN 55133-3427 (US)**

(72) Inventor: **Lindstrom, Kyle J.**
**Saint Paul, MN 55133-3427 (US)**

(74) Representative: **Vossius & Partner**
**Siebertstrasse 4**
**81675 München (DE)**

(56) References cited:
**WO-A-00/76519** **US-A- 6 039 969**

Remarks:
The file contains technical information submitted after the application was filed and not included in this specification

EP 1 719 511 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> Lindstrom, Kyle J.

<120> N-[4-(4-amino-2-ethyl-1H-imidazo[4,5-c]quinolin-1-yl)butyl]methan esulfonamide, a pharmaceutical composition comprising the same and use thereof

<130> K 1722 EP/1

<140> EP 06 01 0882.6
<141> 2002-11-14

<150> US 60/332,412
<151> 2001-11-16

<160> 16

<170> PatentIn version 3.3

<210> 1
<211> 28

<212> DNA
<213> Mus musculus

<400> 1
ctcgagatgg taaagtccct ctgggata 28

<210> 2
<211> 25
<212> DNA
<213> Mus musculus

<400> 2
gtgcacaaag ttcctctcat ggagg 25

<210> 3
<211> 27
<212> DNA
<213> Mus musculus

<400> 3
gtgcacggca agagcattgt ggagaac 27

<210> 4
<211> 29
<212> DNA
<213> Mus musculus

<400> 4
acgcgttcac atcatcctca ttgactaag 29

<210> 5
<211> 29
<212> DNA
<213> Homo sapiens

<400> 5
gcgcgctagc atgaccaaag acaaagaac 29

<210> 6
<211> 30
<212> DNA
<213> Homo sapiens

<400> 6
gcgcgcgtgc acaaagttcc tctcatgaag 30

<210> 7
<211> 33
<212> DNA
<213> Homo sapiens

<400> 7
gcgcgcgtgc acggcaagag cattgtggaa aat 33

<210> 8
<211> 29
<212> DNA
<213> Homo sapiens

<400> 8
acgcgttaag atttcacatc attgttttc 29

<210> 9
<211> 30
<212> DNA
<213> Homo sapiens

<400> 9
gcgcgctagc atggtgtttc caatgtggac 30

<210> 10
<211> 24
<212> DNA
<213> Homo sapiens

<400> 10
ggatccagtc cctttcctcg agac 24

<210> 11
<211> 29
<212> DNA
<213> Homo sapiens

<400> 11
ggatccatgg gcagccagtt ctggaaaac 29

<210> 12
<211> 30
<212> DNA
<213> Homo sapiens

<400> 12
gcgcacgcgt ctagaccgtt tccttgaaca 30

<210> 13
<211> 28
<212> DNA
<213> Mus musculus

<400> 13
ctcgagatgg taaagtccct ctgggata 28

<210> 14
<211> 29
<212> DNA
<213> Mus musculus

<400> 14
acgcgttcac atcatcctca ttgactaag 29

<210> 15
<211> 30
<212> DNA
<213> Mus musculus

<400> 15
cgcgctagca tggtgttttc gatgtggaca 30

<210> 16
<211> 30
<212> DNA
<213> Mus musculus

<400> 16
cgacgcgtgc tagactgttt ccttgaacat 30

SEQUENCE LISTING

<110> Lindstrom, Kyle J.

<120> N- [4- (4-amino-2-ethyl-1H-imidazo[4,5-c] quinolin-1-yl) butyl]methan esulfonamide, a pharmaceutical composition comprising the same and use thereof

<130> K 1722 EP/1

<140> EP 06 01 0882.6
<141> 2002-11-14

<150> US 60/332,412
<151> 2001-11-16

<160> 16

<170> PatentIn version 3.3

<210> 1
<211> 28
<212> DNA
<213> Mus musculus

<400> 1
ctcgagatgg taaagtccct ctgggata 28

<210> 2
<211> 25
<212> DNA
<213> Mus musculus

<400> 2
gtgcacaaag ttcctctcat ggagg 25

<210> 3
<211> 27
<212> DNA
<213> Mus musculus

<400> 3
gtgcacggca agagcattgt ggagaac 27

<210> 4
<211> 29
<212> DNA
<213> Mus musculus

<400> 4
acgcgttcac atcatcctca ttgactaag 29

<210> 5
<211> 29
<212> DNA
<213> Homo sapiens

<400> 5
gcgcgctagc atgaccaaag acaaagaac 29

<210> 6
<211> 30
<212> DNA
<213> Homo sapiens

<400> 6
gcgcgcgtgc acaaagttcc tctcatgaag 30

<210> 7
<211> 33
<212> DNA
<213> Homo sapiens

<400> 7
gcgcgcgtgc acggcaagag cattgtggaa aat 33

<210> 8
<211> 29
<212> DNA
<213> Homo sapiens

<400> 8
acgcgttaag atttcacatc attgttttc 29

<210> 9
<211> 30
<212> DNA
<213> Homo sapiens

<400> 9
gcgcgctagc atggtgtttc caatgtggac 30

<210> 10
<211> 24
<212> DNA
<213> Homo sapiens

<400> 10
ggatccagtc cctttcctcg agac 24

<210> 11
<211> 29
<212> DNA
<213> Homo sapiens

<400> 11
ggatccatgg gcagccagtt ctggaaaac 29

<210> 12
<211> 30
<212> DNA
<213> Homo sapiens

<400> 12
gcgcacgcgt ctagaccgtt tccttgaaca 30

<210> 13
<211> 28
<212> DNA
<213> Mus musculus

<400> 13
ctcgagatgg taaagtccct ctgggata 28

<210> 14
<211> 29
<212> DNA
<213> Mus musculus

<400> 14
acgcgttcac atcatcctca ttgactaag 29

<210> 15
<211> 30
<212> DNA
<213> Mus musculus

<400> 15
cgcgctagca tggtgttttc gatgtggaca 30

<210> 16
<211> 30
<212> DNA
<213> Mus musculus

<400> 16
cgacgcgtgc tagactgttt ccttgaacat 30

**Sequence Listing**

<110> Gorden, Keith
Qiu, Xiaohong
Tomai, Mark
Vasilakos, John

<120> N-[4-(4-amino-2-ethyl-1H-imidazo[4,5-c]quinolin-1-yl)butyl]methanesulfonamide, a pharmaceutical composition comprising the same and use thereof

<130> 7074US010

<140>
<141> [2002-11-14]

<150> 60/332,412

<151> 2001-11-16

<160> 16

<210> 1
<211> 28
<212> DNA
<213> Mus musculus

<400> 1
ctcgagatgg taaagtccct ctgggata

<210> 2
<211> 25
<212> DNA
<213> Mus musculus

<400> 2
gtgcacaaag ttcctctcat ggagg

<210> 3
<211> 27
<212> DNA
<213> Mus musculus

<400> 3
gtgcacggca agagcattgt ggagaac

<210> 4
<211> 29
<212> DNA
<213> Mus musculus

<400> 4
acgcgttcac atcatcctca ttgactaag

<210> 5
<211> 29
<212> DNA
<213> Homo sapiens

<400> 5
gcgcgctagc atgaccaaag acaaagaac

<210> 6
<211> 30
<212> DNA
<213> Homo sapiens

<400> 6
gcgcgcgtgc acaaagttcc tctcatgaag

<210> 7
<211> 33
<212> DNA
<213> Homo sapiens

<400> 7

gcgcgcgtgc acggcaagag cattgtggaa aat

<210> 8
<211> 29
<212> DNA
<213> Homo sapiens

<400> 8
acgcgttaag atttcacatc attgttttc

<210> 9
<211> 30
<212> DNA
<213> Homo sapiens

<400> 9
gcgcgctagc atggtgtttc caatgtggac

<210> 10
<211> 24
<212> DNA
<213> Homo sapiens

<400> 10
ggatccagtc cctttcctcg agac

<210> 11
<211> 30
<212> DNA
<213> Homo sapiens

<400> 11
gcgcacgcgt ctagaccgtt tccttgaaca

<210> 13
<211> 28
<212> DNA
<213> Mus musculus

<400> 13
ctcgagatgg taaagtccct ctgggata

<210> 14
<211> 29
<212> DNA
<213> Mus musculus

<400> 14
acgcgttcac atcatcctca ttgactaag

<210> 15
<211> 30
<212> DNA
<213> Mus musculus

<400> 15
cgcgctagca tggtgttttc gatgtggaca

<210> 16
<211> 30
<212> DNA
<213> Mus musculus

<400> 16
cgacgcgtgc tagactgttt ccttgaacat

**Sequence Listing**

<110> Gorden, Keith
Qiu, Xiaohong
Tomai, Mark
Vasilakos, John

<120> Methods and Compositions related to IRM Compounds and Toll-Like Receptor Pathways

<130> 7074US010

<140>
<141> [2002-11-14]

<150> 60/332,412
<151> 2001-11-16

<160> 16

<210> 1
<211> 28
<212> DNA
<213> Mus musculus

<400> 1
ctcgagatgg taaagtccct ctgggata

<210> 2
<211> 25
<212> DNA
<213> Mus musculus

<400> 2
gtgcacaaag ttcctctcat ggagg

<210> 3
<211> 27
<212> DNA
<213> Mus musculus

<400> 3
gtgcacggca agagcattgt ggagaac

<210> 4
<211> 29
<212> DNA
<213> Mus musculus

<400> 4
acgcgttcac atcatcctca ttgactaag

<210> 5
<211> 29
<212> DNA
<213> Homo sapiens

<400> 5
gcgcgctagc atgaccaaag acaaagaac

<210> 6
<211> 30
<212> DNA
<213> Homo sapiens

<400> 6
gcgcgcgtgc acaaagttcc tctcatgaag

<210> 7
<211> 33
<212> DNA
<213> Homo sapiens

<400> 7
gcgcgcgtgc acggcaagag cattgtggaa aat

<210> 8
<211> 29
<212> DNA
<213> Homo sapiens

<400> 8
acgcgttaag atttcacatc attgttttc

<210> 9
<211> 30
<212> DNA
<213> Homo sapiens

<400> 9
gcgcgctagc atggtgtttc caatgtggac

<210> 10
<211> 24
<212> DNA
<213> Homo sapiens

<400> 10
ggatccagtc cctttcctcg agac

<210> 11
<211> 30
<212> DNA
<213> Homo sapiens

<400> 11
gcgcacgcgt ctagaccgtt tccttgaaca

<210> 13
<211> 28
<212> DNA
<213> Mus musculus

<400> 13
ctcgagatgg taaagtccct ctgggata

<210> 14
<211> 29
<212> DNA
<213> Mus musculus

<400> 14
acgcgttcac atcatcctca ttgactaag

<210> 15
<211> 30
<212> DNA
<213> Mus musculus

<400> 15
cgcgctagca tggtgttttc gatgtggaca

<210> 16
<211> 30
<212> DNA
<213> Mus musculus

<400> 16
cgacgcgtgc tagactgttt ccttgaacat

## Description

Immune response modifiers ("IRMs") include compounds that possess potent immunostimulating activity including but not limited to antiviral and antitumor activity. Certain IRMs effect their immunostimulatory activity by inducing the production and secretion of cytokines such as, e.g., IFN-α, TNF-α, IL-1, IL-6, IL-8, IL-10, IL-12, MIP-1, and MCP-1. Certain IRMs are small organic molecules such as those disclosed in, for example, U.S. Patent Nos. 4,689,338; 4,929,624; 5,266,575; 5,268,376; 5,352,784; 5,389,640; 5,482,936; 5,494,916; 6,110,929; 6,194,425; 4,985,815; 5,175,296; 5,367,076; 5,395,937; 5,693,811; 5,741,908; 5,238,944; 5,939,090; 6,245,776; 6,039,969; 6,083,969; 6,245,776; 6,331,539; and 6,376,669; and PCT Publications WO 00/76505; WO 00/76518; WO, 02/46188, WO 02/ 46189; WO 02/46190; WO 02/46191; WO 02/46192; WO 02/46193 WO 02/46194, and WO 00/76519.

Additional small molecule IRMs include purine derivatives (such as those described in U.S. Patent Nos. 6,376,50 and 6,028,076), small heterocyclic compounds (such as those described in U.S. Patent-No. 6,329,381), and amide derivatives (such as those described in U.S. Patent No. 6,069,149).

Other IRMs include large biological molecules such as oligonucleotide sequences. Some IRM oligonucleotide sequences contain cytosine-guanine dinucleotides (CpG) and are described, for example, in U.S. Patent Nos. 6,1994,388; 6,207,646; 6,239,116; 6,339,068; and 6,406,705. Other IRM nucleotide sequences lack CpG and are described, for example, in International Patent Publication No. WO 00/75304.

By stimulating certain aspects of the immune system, as well as suppressing other aspects (see, e.g., U.S. Patent Nos. 6,039,969 and 6,200,592), IRMs may be used to treat many diseases. For example, the small molecule IRM imiquimod is useful for the treatment of external genital and perianal warts caused by human papillomavirus [Tomai et al., Antiviral Research 28(3): 253-264 (1995)]. Examples of other diseases that may be treated using IRM compounds include, but are not limited to, basal cell carcinoma, eczema, essential thrombocythaemia, hepatitis B, multiple sclerosis, neoplastic diseases, psoriasis, rheumatoid arthritis, type I herpes simplex, and type II herpes simplex.

IRM compounds can modulate cell-mediated immunity by inducing secretion of certain immune system regulator molecules such as cytokines. For example, cytokines that are induced by imiquimod or resiquimod include but are not limited to IFN-α, TNF-α, IL-1, IL-6, IL-8, IL-10, IL-12, MIP-1, and MCP-1 [see, e.g., Tomai et al, Antiviral Research 28 (3): 253-64 (1995); Megyeri et al., Molecular and Cellular Biology 15(4): 2207-18 (1995)].

IRM compounds also can modulate humoral immunity by stimulating antibody production by B cells. Further, various IRMs have been shown to be useful as vaccine adjutants (see, e.g., U.S. Pat. Nos. 6,083,505 and 6,406,705).

Elucidating and differentiating the biological mechanism and signaling pathway underlying the activities of the various FIRM compounds would greatly aid in the identification and development of new IRM compounds and methods of treatment using these compounds.

It has been found that many IRM compounds act through Toll-Like Receptor (TLR) pathways, including pathways mediated by TLR6 and TLR7 .

Disclosed here are methods of identifying an IRM compound that activates a TLR-mediated cellular signaling pathway. The method includes (a) exposing a TLR-positive cell culture to a test compound and measuring a TLR-mediated cellular response; (b) exposing a TLR-negative cell culture to a test compound and measuring a TLR-mediated cellular response; and (c) identifying the test compound as an IRM if the cellular response in the TLR-positive cell culture is greater than the cellular response of the TLR-negative cell culture. In certain embodiments, the methods can identify agonists of TLR6. In other embodiments, the methods can identify agonists of TLR7.

Also disclosed here are methods of identifying an IRM antagonist that inhibits a TLR-mediated cellular signaling pathway. The method includes (a) exposing a first IRM-responsive cell culture to an IRM compound and measuring a TLR-mediated cellular response; (b) exposing a second IRM-responsive cell culture to an IRM compound and a test compound, and measuring a TLR-mediated cellular response; and (c) identifying the test compound as an IRM antagonist if the cellular response in the first cell culture is greater than the cellular response of the second cell culture.

Also disclosed here are compounds identified as TLR agonists, and pharmaceutical compositions that include compounds identified as TLR agonists or pharmaceutically acceptable salts thereof.

Also disclosed are compounds for use in a method of eliciting a TLR-mediated cellular response in a cell that expresses a TLR. The method includes (a) selecting a compound identified as a TLR agonist; and (2) administering to the cell the compound in an amount that affects at least one TLR-mediated cellular signaling pathway. In certain embodiments, the methods include selecting and administering a TLR6 agonist. In other embodiments, the methods include selecting and administering a TLR7 agonist.

Also disclosed here are compounds for use in a method of treating an organism having a condition treatable by modulating a TLR-mediated cellular response. The method includes (a) selecting a compound identified as a TLR agonist; and (b) administering to the organism the compound in an amount effective to modulate a TLR-mediated cellular signaling pathway. In certain embodiments, the methods include selecting and administering a TLR6 agonist. In other embodiments, the methods include selecting and administering a TLR7 agonist.

Reference is made to the following detailed description, examples, claims and appended drawings. In several

places throughout the specification, guidance is provided through lists of examples. In each instance, the recited list serves as a representative group.

Disclosed here are methods of detecting compounds that act as agonists for TLRs. Also disclosed here are methods of identifying compounds that act as antagonists of TLRs. A compound identified as a TLR6 agonist or a TLR7 agonist may be employed to elicit a TLR6-mediated or a TLR7-mediated cellular response, respectively. Such cellular responses include but are not limited to altering cytokine production, NF-κB activation, and expression of co-stimulatory markers. Accordingly, disclosed here are compounds for use in methods of treating an organism having a condition treatable by modulating a TLR6-mediated or TLR7-mediated cellular response. Such conditions include neoplastic diseases, Th1-mediated diseases, Th2-mediated diseases, and infectious diseases (e.g., viral diseases, bacterial diseases, fungal diseases, parasitic diseases, protozoal diseases, prion-mediated diseases, and the like).

For purposes of this invention, the following terms shall have the meanings set forth.

"Agonist" refers to a compound that can combine with a receptor (e.g., a TLR) to produce a cellular response. An agonist may be a ligand that directly binds to the receptor. Alternatively, an agonist may combine with a receptor indirectly by, for example, (a) forming a complex with another molecule that directly binds to the receptor, or (b) otherwise resulting in the modification of another compound so that the other compound directly binds to the receptor. An agonist may be referred to as an agonist of a particular TLR (e.g., a TLR6 agonist).

"Cellular signaling pathway" refers to a cascade of biochemical activity that biochemically links an agonist-receptor interaction with a cellular response to the agonist-receptor binding (e.g., cytokine production).

"Dominant negative" refers to a variant of a naturally occurring protein in which the variant has been altered to possess at least one natural activity, but lack at least one other natural activity. As a nonlimiting example, a dominant negative variant of a receptor protein may bind to its normal binding partner (e.g., a ligand) but fail to promote a second activity that normally results from the receptor-ligand binding (e.g., relay a cellular signal).

"Express/expression" refers to the ability of a cell to transcribe a structural gene, resulting in an mRNA, then translating the mRNA to form a protein that provides a detectable biological function to the cell.

"Inhibit" refers to any measurable reduction of biological activity. Thus, as used herein, "inhibit" or "inhibition" may be referred to as a percentage of a normal level of activity.

"Imiquimod" refers to 1-(2-methylpropyl)-1H-imidazo[4,5-c]quinolin-4-amine.

"IRM antagonist" refers to any compound that inhibits biological activity that normally results from exposing an IRM-responsive cell to an IRM compound.

"IRM compound" refers to a compound that alters the level of one or more immune regulatory molecules, e.g., cytokines or co-stimulatory markers, when administered to an IRM-responsive cell. Representative IRM compounds include the small organic molecules, purine derivatives, small heterocyclic compounds, amide derivatives, and oligonucleotide sequences described above.

"IRM-responsive cell" refers to any cell that exhibits a cellular response when exposed to an IRM compound.

"Resiquimod" refers to 4-amino-2-ethoxymethyl-α,α-dimethyl-1H-imidazo[4,5-*c*]quinoline-1-ethanol.

"TLR-mediatd" refers to a biological or biochemical activity that results from TLR function. A particular biological or biochemical activity may be referred to as mediated by a particular TLR (e.g., "TLR6-mediated" or "TLR7-mediated").

"TLR-positive" refers to a cell culture selected to provide greater detectable function of a particular TLR (e.g., "TLR6-positive" or TLR7-positive") than a corresponding TLR-negative cell culture (e.g., "TLR6-negative" or "TLR7-negative"). A TLR-positive cell culture may exhibit greater than normal TLR function, e.g., overexpression of TLR function compared to a TLR-negative cell culture exhibiting generally normal TLR function. Alternatively, a TLR-positive cell culture may exhibit generally normal or less than normal TLR function, e.g., a cell culture exhibiting generally normal TLR function compared to a TLR-negative cell culture exhibiting inhibited TLR function.

"TLR-negative" refers to a cell culture selected to provide less detectable function of a particular TLR (e.g., "TLR6-negative" or "TLR7-negative") than a corresponding TLR-positive cell culture (e.g., "TLR6-positive" or TLR7-positive"). A TLR-negative cell culture may exhibit less than normal TLR function, e.g., inhibited TLR function compared to a TLR-positive cell culture exhibiting generally normal TLR function. Alternatively, a TLR-negative cell culture may exhibit generally normal or greater than normal TLR function, e.g., a cell culture exhibiting generally normal TLR function compared to a TLR-positive cell culture exhibiting greater than normal TLR function.

Certain cells of the immune system (e.g., antigen presenting cells, or "APCs") recognize foreign antigens, some of which potentially may be harmful to the host, and trigger an immune response against the antigens Toll-Like Receptors (TLRs) are a family of immune system receptors that permit cells of the immune system to recognize specific molecular patterns presented by foreign antigens. The molecular patterns are commonly termed pathogen-associated molecular patterns ("PAMPs"). The TLRs include an extracellular domain that contains a leucine-rich domain and a cytoplasmic domain that resembles the cytoplasmic-domain of the interleukin-1 receptor.

Activation of the various TLRs induces a range of biological effects including the secretion of cytokines and antimicrobial peptides. Cytokines are important immune system regulatory molecules and include, but are not limited to, TNF-α, IFN-α, and the interleukins. Cytokines act upon cellular receptors and regulate such diverse cellular activities

as cell growth, cell differentiation, cell death, the inflammatory process, and cell migration.

The discovery of different TLRs has led to the identification of signaling pathways that connect the receptors to the biological effects of their activation. The cytoplasmic protein MyD88 has been identified as one member of cellular signaling pathways that also include various TLRs. The MyD88 protein has an IL-1 receptor domain similar to that of the cytoplasmic domain of the TLRs. The IL-1 receptor domain of the MyD88 and the cytoplasmic TLR domain interact when the TLR binds to a ligand and, in turn, cause other cytoplasmic proteins (e.g., IRAK and TRAF6) to interact. The signal cascade that begins with an agonist binding to a TLR and is relayed through IRAK and TRAF6 eventually activates NF-κB, which stimulates transcription of various genes including those encoding cytokines such as TNF-α, IL-6, and IL-12.

Many IRM compounds share a number of cellular activities, many of which are conserved across species, e.g., upregulation of co-stimulatory markers, induction of pro-inflammatory cytokines in monocyte/macrophage cells, and activation of transcriptional regulators NF-κB and AP-1. Identifying TLR agonists, including but not limited to IRM compounds, also may identify compounds having prophylactic or therapeutic utility for certain conditions that are treatable by inducing an immune response through one or more TLRs.

A dominant-negative variant of a TLR may be employed to identify agonists of the TLRs. Table 2 shows how the use of a dominant negative variant of TLR6 (TLR6DN) or TLR7 (TLR7DN) may be used to identify an agonist of TLR6 or. TLR7, respectively. Two sets of THP-1 cells were transfected with a vector into which construct encoding a dominant-negative variant of a TLR (generally, TLRDN) had been cloned. One set of cells was transfected with vector including a TLR6DN construct; the other set was transfected with vector including a TLR7DN construct. THP-1 cells are human monocyte cells derived from acute monocytic leukemia tissue and are known to exhibit increased TNF-α production upon stimulation with TLR agonists such as zymosan (a know agonist of TLR6) or LPS (a known agonist of TLR4). As a control, THP-1 cells were also transfected with vector lacking a dominant-negative TLR construct.

The transfectants were cultured and exposed to various stimuli: LPS, zymosan, and resiquimod, an IRM compound. The effect of the dominant-negative variants was assessed by measuring the extent to which TNF-α production, upon exposure to a stimulus, was inhibited in cells transfected with a TLRDN compared to cells transfected with a control vector. TLR6DN inhibited TNF-α production upon stimulation with zymosan - a known TLR6 - agonist - and resiquimod, but did not materially inhibit TNF-α production when stimulated with the TLR4 agonist LPS. TLR7DN inhibited TNF-α production upon stimulation with LPS and resiquimod, but did not materially inhibit TNF-a production upon stimulation with zymosan.

Table 3 illustrates that the effect is not specific to the host cell type. The TLR6DN construct was transfected into RAW 264.7 cells, a mouse macrophage cell line known to produce TNF-α upon stimulation with a TLR agonist, such as zymosan or LPS. As in the THP-1 cells, TNF-α production by TLR6DN-transfected RAW 264.7 cells was inhibited to a much greater extent when upon stimulation with zymosan or resiquimod than when stimulated with the TLR7 agonist LPS.

Thus, a dominant negative variant of a TLR may be employed to identify an agonist of the TLR. The use of TLR6DN can be used to confirm that a known TLR6 agonist, such as zymosan, acts through TLR6. TLR6DN also can be used to identify additional TLR6 agonists, such as IRM compounds including but not limited to resiquimod. Similarly, TLR7DN may be used to confirm that a known TLR7 agonist acts through TLR7. TLR7DN also can be used to identify additional TLR7 agonists, such as IRM compounds including but not limited to resiquimod. One skilled in the art will recognize that a broad range of potential IRM compounds may be screened in this fashion to identify agonists of any TLR for which a TLRDN can be constructed and expressed.

A TLR agonist also can be identified by employing TLR-specific antibodies that neutralize TLR function. Table 4 shows that anti-TLR6 antibodies can be used to specifically inhibit TLR6-mediated TNF-α production. When RAW 264.7 cells are preincubated with anti-TLR6 antibodies and then incubated with various stimuli, the TNF-α production induced by known TLR6 agonists peptidoglycan and zymosan is inhibited by the antibodies to a greater extent than TNF-α production in response to the TLR4 agonists LPS. In addition, stimulation of TNF-α production by various IRM compounds also is strongly inhibited by presence of the anti-TLR6 antibodies, thereby identifying these IRM compounds as TLR6 agonists.

Overexpression of a TLR also can be used to identify a TLR agonist. Table 5 shows that overexpression of TLR6 or TLR7 can make RAW 264.7 cells more sensitive to IRM induction of TNF-α production. Specifically, RAW 264.7 cells can be transfected with a vector that encodes a TLR (e.g., TLR6 or TLR7) expressed from a strung eukaryotic promoter. When incubated with various concentrations of resiquimod, the RAW 264.7 cells can exhibit increased stimulation of TNF-α production compared to resiquimod-stimulated untransfected RAW 264.7 cells. For both cultures of TLR-overexpression transfectants, the extent to which TNF-α production is stimulated decreases as the concentration of resiquimod increases (i.e., the dose-response curve was shifted lower). Thus, resiquimod is an agonist of each of TLR6 and TLR7: The data also show that, in a given cell, the induction of TNF-α production by resiquimod is limited by the extent to which the cell expresses TLR.

Table 6 shows that a broad spectrum of IRM compounds can induce NF-κB activation through TLR7. HEK293 cells, derived from human embryonic kidney cells, may be co-transfected with (1) either a control vector or a vector

construct including human TLR7, and (2) an NF-κB-luciferase reporter. The NF-κB-luciferase reporter provides a luciferase signal upon NF-κB activation in a transfected cell. Thus, TLR7-mediated NF-κB activity can be detected by exposing the cells transfected with vector and the cells transfected with the TLR7 construct to an IRM compound, then comparing the luciferase signal of the vector-transfected cells with the luciferase signal of the cells transfected with the TLR7 construct.

Table 6 shows that various IRM compounds stimulate NF-κB activity in transfected cells to varying degrees, ranging up to more than an 12-fold increase in NF-κB activation over cells transfected with only vector.

**Assays**

The present invention provides assays that can be used to discover new IRM compounds that can activate or inhibit at least one Toll pathway. The assays described below are hot embodiments of the invention.

Disclosed here are methods for identifying an IRM compound that activates at least one Toll pathway, wherein the methods include determining whether a particular compound elicits a TLR-mediated cellular response. One way this can be done is by eliminating or reducing the activity of at least one TLR in a cell and measuring the resulting effect of eliminating the TLR on at least one TLR-mediated cellular response.

The methods include transfecting an IRM-responsive cell with a dominant-negative variant of a TLR to eliminate or to measurably reduce TLR-mediated activity upon exposure of the transfected cell to an IRM compounds.

A dominant-negative variant (TLRDN) can be constructed in various ways. In some embodiments, a TLRDN can be made by altering the cytoplasmic domain of the protein, thereby disrupting binding between the TLR and its cytoplasmic binding partners. In other embodiments, the TLR may be altered to disrupt TLR-agonist binding. Regardless of the specific change made in the TLR, a dominant-negative variant will be unable to relay at least one TLR-mediated cellular signal when exposed to a TLR agonist.

A mutation resulting in a TLRDN may be a point mutation, a. deletion or an insertion. A deletion or insertion may be of any size. In some of these embodiments, the mutation can be non-conservative. In other embodiments, the mutation can be conservative. In yet other embodiments, the mutation at the DNA level may form a stop codon, resulting in a truncated protein. Alternatively, the mutation may cause a shift in the reading frame that changes the amino acid sequence downstream from the frameshift mutation.

One method of identifying an IRM compound that activates a TLR-mediated cell signaling pathway includes exposing a TLR-positive cell culture to a test compound and measuring a TLR-mediated cellular response; exposing a TLR-negative cell culture to a test compound and measuring a TLR-mediated cellular response; and identifying the compound as an IRM compound if the cellular response in the TLR-positive cell culture is greater than the cellular response of the TLR-negative cell culture.

The step of exposing a TLR-positive cell culture to a test compound and measuring a TLR-mediated cellular response may include exposing a control IRM-responsive cell culture (e.g., cells transfected with a null vector) to the test compound, measuring the TLR-mediated cellular response of the control culture, and comparing the cellular response of the TLR-positive test culture to the cellular response of the control culture. Similarly, the step of exposing a TLR-negative cell culture to a test compound and measuring a TLR-mediated cellular response may include exposing a control IRM-responsive cell culture to the test compound, measuring the TLR-mediated cellular response in the control culture, and comparing the cellular response of the TLR-negative test culture to the cellular response of the control culture. However, with experience, one skilled in the art may develop sufficient familiarity with a particular assay that explicit use of controls may not always be necessary to identify an IRM compound using the methods of the present invention.

The method may be designed to identify compounds that activate any particular TLR. Routine methods may be employed to produce a TLR-positive cell culture, a TLR-negative cell culture, or both for any particular TLR. The method may be designed to identify a compound that activates a TLR6-mediated cell signaling pathway. The method may be designed to identify a compound that activates a TLR7-mediated cell signaling pathway.

The TLR-positive cell culture may include cells that provide a greater than normal IRM-mediated cellular response. For example, the TLR-positive cell culture may include cells that have been genetically modified, such as by transfection, to provide a greater than normal IRM-mediated response when stimulated with an IRM. Such genetic modifications may include providing additional copies of TLR structural genes so that transfected cells overexpress the TLR. Additionally, overexpression of a TLR may result from cloning the relevant TLR gene under the control of one or more strong transcriptional regulatory sequences.

The TLR-positive cell culture may include transfected cells that overexpress TLR6. Alternatively, the TLR positive cell culture may include cells transfected to overexpress TLR7. Cells that express or overexpress a TLR can be made by various standard techniques (See, e.g., Current Protocols in Molecular Biology, John Wiley and Sons, Inc. (2001)). In embodiments in which the TLR-positive cell culture provides a greater than normal TLR-mediated cellular response, the TLR-negative cell culture may includes cells that provide a generally normal level TLR-mediated cellular

response: Alternatively, the TLR-negative cell cultures may include cells that provide a lower than normal TLR-mediated cellular response.

Alternatively, the TLR-positive cell culture may include cells that provide a generally cellular response. The TLR-negative cell culture includes cells that provide a lower than normal TLR-mediated cellular response. The TLR-negative cell culture may include cells that have been genetically modified to provide the lower than normal TLR-mediated response when stimulated with an IRM. For example, the TLR-negative cell culture may include cells that have been transfected with a vector that encodes a dominant-negative TLR variant including but not limited to TLR6DN and TLR7DN. Alternatively, the TLR-negative cell culture may include cells that have been transfected with vectors that include antisense constructs of a TLR to at least partially inhibit expression of the TLR. See, e.g., Current Protocols in Molecular Biology, John Wiley and Sons, Inc. (2001).

Alternatively, the TLR-negative cell culture may include one or more inhibitory components that interfere with either (1) binding of the test compound with the TLR, or (2) the ability of the TLR to relay a cellular signal after binding to an agonist (i.e., the test compound). For example, the TLR-negative cell culture may include an antibody that specifically binds to the TLR (an anti-TLR antibody, generally), thereby at least partially inhibiting the TLR-mediated cellular response. The generation of an antibody that specifically binds to a particular target is considered routine to one skilled in the art. Thus, an anti-TLR antibody can be used to provide a TLR-negative cell culture according to the methods of the present invention. Alternatively, an anti-TLR6 antibody may be used to provide a TLR6-negative cell culture. The anti-TLR antibody may be added to the cell culture prior to the test compound or may be added with the test compound. The anti-TLR antibody may be polyclonal or monoclonal. The final ' concentration of antibody in the cell culture may range from about 0.01 μg/ml to about 100 μg/ml. The cells of the cell culture may be pre-incubated with the anti-TLR antibody from about 0 minutes to about 48 hours prior to addition of the test compound.

The TLR-mediated cellular response may include Production of at least one cytokine including TNF-α, IFN-α, IL-1, IL-6, IL-8, IL-10, IL-12, MIP-1, MCP-1, or any combination thereof. The TLR-mediated cellular response may include activation of NF-κB. The TLR-mediated cellular response may include production of one or more co-stimulatory markers including CD40, CD80, CD86, and CCR7.

Also disclosed here are methods for identifying IRM compounds that activate at least one TLR-mediated cellular signaling pathway, wherein the methods comprise the use of TLR deficient mice (knockout mice). With knockout mice, the IRM compounds can be identified by their effects at the whole organism level. Techniques for generating such mice are well-established in the art, and one of skill in the art would readily be able to create such mice See, e.g., Current Protocols in Molecular Biology, John Wiley and Sons, Inc. (2001). Alternatively, specific knockout mice can be ordered custom-made from various commercial services such as inGenious Targeting Laboratory, Inc. (Stony Brook, NY).

When using TLR6 and/or TLR7 knockout mice, the compound may be administered to the mouse and, after a suitable incubation period, the effects on the mouse may be analyzed. The effects may be analyzed, in certain of these embodiment, by measuring cytokine levels from the blood of the treated mice. Certain cell types may be isolated from the treated mice and the production of cytokines or NF-κB activation determined by known methods.

Typically, cells in which TLR6 and/or TLR7 expression has been at least partially inhibited will exhibit at least a 20% reduction in the extent to which administration of the IRM compound stimulates IRM-mediated activity (e.g., cytokine production or NF-κB activation) compared to untransfected cells stimulated with the same concentration of test compound. The cells may exhibit at least a 50% reduction in the extent to which administration of an IRM stimulates IRM-mediated activity. In other embodiments, at least an 80% reduction is observed.

As indicated above, the methods disclosed herein may be employed to identify agonists of any desired TLR. One of ordinary skill in the art can create a TLR-positive cell culture or a TLR-negative cell culture for any particular TLR using the methods described above.

The method may be designed to identify an agonist of TLR6 by employing a TLR6 overexpression cell culture as a TLR6-positive cell culture, an unmodified cell culture as a TLR6-negative cell culture, and measure a TLR6-mediated cellular response in each cell culture after stimulation with a test compound. In an alternative embodiment identifying a TLR-6 agonist, the method may employ an unmodified cell culture as a TLR6-positive cell culture, and either a TLR6DN cell culture or a cell culture that includes anti-TLR6 antibodies as the TLR6-negative cell culture.

The method may be designed to identify an agonist of TLR7 by employing a TLR7 overexpression cell culture as a TLR7-positive cell culture, an unmodified cell culture as a TLR7-negative cell culture, and measure a TLR7-mediated cellular response in each cell culture after stimulation with a test compound. In an alternative embodiment identifying a TLR-7 agonist, the method may employ an unmodified cell culture as a TLR7-positive cell culture, and either a TLR7DN cell culture or a cell culture that includes anti-TLR7 antibodies as the TLR7-negative cell culture.

The present invention also discloses compounds identified as IRM compounds based on the character of the compound as an agonist of a TLR. The compounds disclosed herein are agonists of TLR6 or agonists of TLR7. The present invention also discloses pharmaceutical compositions that include a compound that is a TLR agonist, or pharmaceutically acceptable salts of TLR agonist compounds. Pharmaceutical compositions may include one or more additional components including a pharmaceutically acceptable vehicle, one or more adjuvants, one or more pharmaceutically active compounds (i.e., the TLR agonists may serve as an adjuvant).

Also disclosed are methods of identifying an IRM antagonist that inhibits a TLR-mediated cellular signaling pathway. Such methods include exposing a first IRM-responsive cell culture to an IRM compound and measuring an IRM-mediated cellular response; exposing a second IRM-responsive cell culture to an IRM compound and a test compound and measuring an IRM-mediated cellular response; and identifying the test compound as an IRM antagonist if the cellular response in the first cell culture is greater than the cellular response in the second cell culture.

The IRM-responsive cell culture may include cells that naturally express one or more TLRs. Alternatively, the IRM-responsive cell culture may include cells of any of the IRM-positive cell cultures described above. An antagonist of IRM that is an agonist of a particular TLR may be identified by employing a particular TLR-positive cell culture in the present method. For example, an antagonist of a TLR7 agonist IRM may be identified using a TLR7-positive cell culture such as a cell culture including cells designed to overexpress TLR7 when exposed to an IRM compound.

As with the identification methods described above, the identification of IRM antagonist compounds may include the use of a control cell culture against which the TLR-mediated cellular response of the first IRM-responsive cell culture and second IRM-responsive cell culture are compared. However, again similar to the methods described above, one skilled in the art may develop sufficient familiarity with the assay that running a control for each assay may become unnecessary.

The concentration of the test compound being assayed by the above methods may range from about 0.001 μM to about 100 μM. The cell culture may be incubated with the test compound from about 10 minutes to about 24 hours. The density of cells incubated with the compound to be tested may be from 1 x $10^4$ to 1 x $10^7$ cells/ml.

Cytokine levels are determined using a commercially available ELISA assay. Alternatively, cytokine levels are determined using such techniques as antibody detection and quantitation (*e.g.*, flow cytometry, western blotting, immunohisto/cytochemistry), and bioassays (*e.g.*, L929 cytotoxicity essay where the amount of cell death is directly proportional to the amount of TNF-α in the sample). See, e.g., Current Protocols in Immunology, John Wiley and Sons, Inc. (2001).

The cytokine that is assayed can be TNF-α. TNF-α levels can be determined by ELISA assay. As the minimum level of detection for this assay is 40-80 pg/ml, the test is considered suspect if the level of TNF-α following stimulation is under 100 pg/ml, and the experiment should be redone

IRM-responsive cells used in the above-described methods may be from plants or from animals, particularly vertebrate organisms. The IRM-responsive cells may be from mammals such as, but not limited to, human, rodents dog, cat, sheep, cow, or rabbit. These IRM-responsive cells may include, but are not limited to, monocytes, macrophages; Langerhans cells, dendritic cells, and B-cells. The IRM-responsive cells may be from established cell lines such as RAW 264.7, THP-1,or HEK293.

The TLR genes utilized in the methods may derive from a variety of plant and animal sources including mammals such as, but not limited to, human, rodent, dog, cat, sheep, cow, or rabbit.

The expression of a particular TLRs in cells employed in the methods of the present invention may result from natural gene expression in the cells. Cells that naturally express TLRs include RAW 264.7 cells, THP-1 cells, HEK293 cells, monocytes, dendritic cells, macrophages, and B lymphocytes. Alternatively, the expression of a particular TLR may result from the genetic modification of cells. The cells so modified may naturally express or they may lack natural expression of the particular TLR. The expression of a particular TLR in cells employed in the methods of the present invention may be at a level higher than, lower than, similar to, or equal to the normal level of expression of the particular TLR in the particular line of cells.

Many different cytokines and/or co-stimulatory markers can be assayed in the methods described above. Suitable measurable cytokines include, but are not limited to, TNP-α, IFN-α, IL-1, IL-6, IL-8, IL-10, IL-12, MIP-1, and MCP 1. Suitable measurably co-stimulatory markers include CD40, CD80, CD86 and CCR7.

A compound identified as a TLR agonist or a TLR antagonist by any of the methods described above, or identified by any other method, may be employed to elicit TLR-mediated cellular responses. As used herein, the term "elicit" includes upregulation or downregulation of a particular cellular response. A compound identified as a TLR agonist or a TLR antagonist by any of the methods described above, or identified by any other methods, also may be used to treat an organism having a condition treatable by modulating a TLR-mediated cellular response.

**Methods for Eliciting TLR-Mediated Cellular Responses**

Described herein are methods of eliciting a TLR-mediated cellular response by manipulating a TLR-mediated signaling pathway. Certain TLR-mediated cellular responses elicited by the methods described herein include induction of cytokine production; other cellular responses include inhibiting production of certain cytokines.

Also described is a method of eliciting at least one TLR-mediated cellular response in an IRM-responsive cell by administering to the IRM-responsive cells an IRM compound that affects at least one TLR-mediated cellular signaling pathway

The IRM compound may be any suitable IRM compound. In certain embodiments, suitable IRM compounds

include imidazopyridine amines; imidazonaphthyridine amines; imidazotetrahydronaphthyridine amines; thiazoloquinoline amines; thiazolonaphthyridine amines; imidazothienopyridines; oxazoloquinoline amines; or imidazoquinoline amines including but not limited to 1,2-bridged imidazoquinoline amines, sulfonamido-substituted imidazoquinoline amines; urea-substituted imidazoquinoline amines; or heteroaryl ether-substituted imidazoquinoline amines. Specifically, suitable IRM compounds include *N*-[4-(4-amino-2-butyl-6,7-dimethyl-1*H*-imidazo[4,5-*c*]pyridin-1-yl)butyl]methanesulfonamide; *N*-[4-(4-amino-2-butyl-1*H*-imidazo[4,5-*c*]quinolin-1-yl)butyl]methanesulfonamide; 1-{2-[3-(3-pyridyl)propoxy]ethyl}-1*H*-imidazo[4,5-*c*]quinolin-4-amine; 4-amino-2-butyl-α,α-dimethyl-1*H*-imidazo[4,5-*d*]thieno[3,2-*b*]pyridine-1-ethanol; 2-butyl-6,7,8,9-tetrahydro-1-(2-methylpropyl)-1*H*-imidazo[4,5-*c*][1,5]naphthyridin-4-amine; *N*-[4-(4-amino-2-ethyl-1*H*-imidazo[4,5-*c*]quinolin-1-yl)butyl]methanesulfonamide (the compound of claim 1); or 4-amino-2-(ethoxymethyl)-α,α-dimethyl-6,7,8,9-tetrahydro-1*H*-imidazo[4,5-*c*]quinolin-1-ethanol hydrate.

Suitable IRM compounds also include the purine derivatives, small heterocyclic compounds, amide derivatives, and oligonucleotide sequences described above.

The TLR-mediated cellular response may include production of at least one cytokine including TNF-α, IFN-α, IL-1, IL-6, IL-8, IL-10, IL-12, MIP-1, MCP-1, or any combination thereof. The TLR-mediated cellular response may include activation of NF-κB. Alternatively, the TLR-mediated cellular response may include production of one or more co-stimulatory markers including, but not limited to, CD40, CD80, CD86 and CCR7. Suitable IRM-responsive cells include monocytes, macrophages, Langerhans cells, dendritic cells, and B lymphocytes.

**Treatments**

The activation of a TLR pathway of an organism may result in increased or decreased production of at least one cytokine. Because the ability to control cytokine levels can be useful in the treatment of cytokine-related conditions, the present invention also provides the compound of claim 1 for treating these conditions. It is possible that in certain embodiments, production of one or more cytokines will be induced, while the production of one or more other cytokines will be inhibited.

Therefore, the present invention provides a compound for treating an organism having a condition treatable by modulating a TLR-mediated cellular response. The IRM compound may be an agonist of any suitable TLR (e.g., TLR6 or TLR7).

Activation of a TLR pathway may be useful in treating a variety of disorders that are responsive to cytokines. Activation of a TLR pathway may have an effect on the acquired immune response. For example, the production of the T helper type 2 (Th2) cytokines IL-4, IL-5 and IL-13 are inhibited upon activation of the TLR pathway. This activity indicates that the methods of the present invention may provide treatment of conditions where upregulation of the Th1 response and/or down regulation of the Th2 response is desired. Such conditions include atopic diseases (e.g., atopic dermatitis, asthma, allergy, allergic rhinitis) and systemic lupus erythematosis. The compounds disclosed herein also may provide vaccine adjuvants for cell mediated immunity and treatments for recurrent fungal diseases and chlamydia.

Agents that activate the TLR pathway are expected to be particularly useful in the treatment of viral diseases and tumors. Their immunomodulating activity suggests that such agents are useful in treating diseases including, but not limited to, viral diseases including genital warts, common warts, plantar warts, Hepatitis B, Hepatitis C, Herpes Simplex Virus Type I and Type II, rhinovirus, adenovirus, influenza, para-influenza, molluscum contagiosum, varriola major, HIV, CMV, VZV; intraepithelial neoplasias such as cervical intraepithelial neoplasia, human papillomavirus (HPV), and associated neoplasias; fungal diseases, e.g., candida, aspergillus, onychomycosis, tinea pedia, and cryptococcal meningitis; neoplastic diseases, e.g., basal cell carcinoma, hairy cell leukemia, Kaposi's sarcoma, renal cell carcinoma, squamous cell carcinoma, myelogenous leukemia, multiple myeloma, melanoma, non-Hodgkin's lymphoma, cutaneous T-cell lymphoma, and other cancers; parasitic diseases, e.g., pneumocystis carnii, cryptosporidiosis, histoplasmosis, toxoplasmosis, trypanosome infection, and leishmaniasis; and bacterial infections, e.g., tuberculosis, and mycobacterium avium. Additional diseases or condition that can be treated using agents that activate the TLR pathway include actinic keratosis, eczema, eosinophilia, essential thrombocythaemia, leprosy, multiple sclerosis, Ommen's syndrome, discoid lupus, Bowen's disease, Bowenoid papulosis, and alopecia areata. In addition, such agents could inhibit formation of Keloids and other types of post-surgical scars and enhance or stimulate the healing of wounds, including chronic wounds. The agents may be useful for treating the opportunistic infections and tumors that occur after suppression of cell mediated immunity in, for example, transplant patients, cancer patients and HIV patients.

Suitable IRM compound can be a known IRM compound including the small organic IRM molecule described in detail below, or the purine derivatives, small heterocyclic compounds, amide derivatives, and oligonucleotide sequences described above.

An amount of an IRM compound or other agent effective to activate the Toll pathway and induce cytokine biosynthesis is an amount sufficient to cause one or more cell types, such as monocytes, macrophages, dendritic cells and B-cells to produce an amount of one or more cytokines such as, for example, IFN-α, TNF-α, IL-1, IL-6, IL-10 and IL-12 that is increased over the background level of such cytokines. The precise amount will vary according to factors

known in the art but is expected to be a dose of about 100 ng/kg to about 50 mg/kg, preferably about 10 μg/kg to about 5 mg/kg. IRM compounds are the preferred agent for activation of the TLR pathway.

The organism treated for the disorder may be a plant or animal, particularly a vertebrate. Preferably the organism treated for the disorder is a mammal, such as, but not limited to, human, rodent, dog, cat, pig, sheep, goat, or cow.

**IRM Compounds**

Known IRM compounds which do not form part of the present invention include 1*H*- imidazo[4,5-*c*]quinolin-4-amines defined by one of Formulas I-V below:

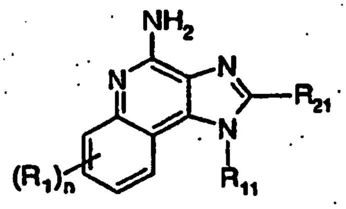

I

wherein

$R_{11}$ is selected from the group consisting of alkyl of one to ten carbon atoms, hydroxyalkyl of one to six carbon atoms, acyloxyalkyl wherein the acyloxy moiety is alkanoyloxy of two to four carbon atoms or benzoyloxy, and the alkyl moiety contains one to six carbon atoms, benzyl, (phenyl)ethyl and phenyl, said benzyl, (phenyl)ethyl or phenyl substituents being optionally substituted on the benzene ring by one or two moieties independently selected from the group consisting of alkyl of one to four carbon atoms, alkoxy of one to four carbon atoms and halogen, with the proviso that if said benzene ring is substituted by two of said moieties, then said moieties together contain no more than six carbon atoms;

$R_{21}$ is selected from the group consisting of hydrogen, alkyl of one to eight carbon atoms, benzyl, (phenyl)ethyl and phenyl, the benzyl, (phenyl)ethyl or phenyl substituent being optionally substituted on the benzene ring by one or two moieties independently selected from the group consisting of alkyl of one to four carbon atoms, alkoxy of one to four carbon atoms and halogen, with the proviso that when the benzene ring is substituted by two of said moieties, then the moieties together contain no more than six carbon atoms; and

each $R_1$ is independently selected from the group consisting of alkoxy of one to four carbon atoms, halogen, and alkyl of one to four carbon atoms, and n is an integer from 0 to 2, with the proviso that if n is 2, then said $R_1$ groups together contain no more than six carbon atoms;

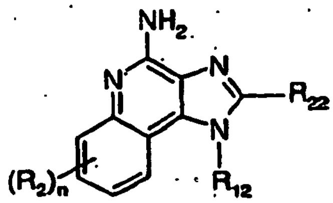

II

wherein

$R_{12}$ is selected from the group consisting of straight chain or branched chain alkenyl containing two to ten carbon atoms and substituted straight chain or branched chain alkenyl containing two to ten carbon atoms, wherein the substituent is selected from the group consisting of straight chain or branched chain alkyl containing one to four carbon atoms and cycloalkyl containing three to six carbon atoms; and cycloalkyl containing three to six carbon atoms substituted by straight chain or branched chain alkyl containing one to four carbon atoms; and

$R_{22}$ is selected from the group consisting of hydrogen, straight chain or branched chain alkyl containing one to eight carbon atoms, benzyl, (phenyl)ethyl and phenyl, the benzyl, (phenyl)ethyl or phenyl substituent being optionally substituted on the benzene ring by one or two moieties, independently selected from the group consisting of straight ; chain or branched chain alkyl containing one to four carbon atoms, straight chain or branched chain alkoxy containing one to four carbon atoms, and halogen, with the proviso that when the benzene ring is substituted by two such moieties, then

the moieties together contain no more than six carbon atoms; and
each $R_2$ is independently selected from the group consisting of straight chain or branched chain alkoxy containing one to four carbon atoms, halogen, and straight chain or branches chain alkyl containing one to four carbon atoms, and n is an integer from zero to 2, with the proviso that if n is 2, then said $R_2$ groups together contain no more than six carbon atoms;

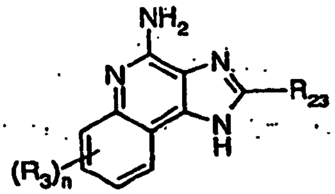

wherein
$R_{23}$ is selected from the group consisting of hydrogen, straight chain or branched chain alkyl of one to eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl of one to four carbon atoms, straight chain or branched chain alkoxy of one to four carbon atoms, and halogen, with the proviso that when the benzene ring is substituted by two such moieties, then the moieties together contain no more than six carbon atoms; and
each $R_3$ is independently selected from the group consisting of straight chain or branched chain alkoxy of one to four carbon atoms, halogen, and straight chain or branched chain alkyl of one to four carbon atoms, and n is an integer from zero to 2, with the proviso that if n is 2, then said $R_3$ groups together contain no more than six carbon atoms;

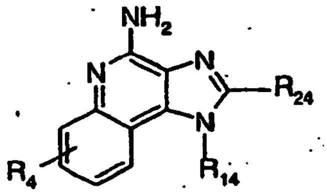

IV

wherein
$R_{14}$ is $-CHR_xR_y$ wherein $R_y$ is hydrogen or a carbon-carbon bond, with the proviso that when $R_y$ is hydrogen $R_x$ is alkoxy of one to four carbon atoms, hydroxyalkoxy of one to four carbon atoms, 1-alkynyl of two to ten carbon atoms, tetrahydropyranyl, alkoxyalkyl wherein the alkoxy moiety contains one to four carbon atoms and the alkyl moiety contains one to four carbon atoms, 2-, 3-, or 4-pyridyl, and with the further proviso that when $R_y$ is a carbon-carbon bond $R_y$ and $R_x$ together form a tetrahydrofuranyl group optionally substituted with one or more substituents independently selected from the group consisting of hydroxy and hydroxyalkyl of one to four carbon atoms;
$R_{24}$ is selected from the group consisting of hydrogen, alkyl of one to four carbon atoms, phenyl, and substituted phenyl wherein the substituent is selected from the group consisting of alkyl of one to four carbon atoms, alkoxy of one to four carbon atoms, and halogen; and
$R_4$ is selected from the group consisting of hydrogen, straight chain or branched chain alkoxy containing one to four carbon atoms, halogen, and straight chain or branched chain alkyl containing one to four carbon atoms;

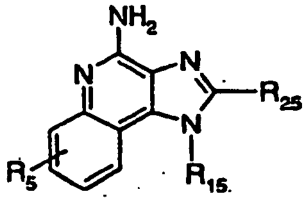

V

wherein

$R_{15}$ is selected from the group consisting of: hydrogen; straight chain or branched chain alkyl containing one to ten carbon atoms and substituted straight chain or branched chain alkyl containing one to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; straight chain or branched chain alkenyl containing two to ten carbon atoms and substituted straight chain or branched chain alkenyl containing two to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; hydroxyalkyl of one to six carbon atoms; alkoxyalkyl wherein the alkoxy moiety contains one to four carbon atoms and the alkyl moiety contains one to six carbon atoms; acyloxyalkyl wherein the acyloxy moiety is alkanoyloxy of two to four carbon atoms or benzoyloxy, and the alkyl moiety contains one to six carbon atoms; benzyl; (phenyl)ethyl; and phenyl; said benzyl, (phenyl)ethyl or phenyl substituent being optionally substituted on the benzene ring by one or two moieties independently selected from the group consisting of alkyl of one to four carbon atoms, alkoxy of one to four carbon atoms, and halogen, with the proviso that when said benzene ring is substituted by two of said moieties, then the moieties together contain no more than six carbon atoms;

$R_{25}$ is

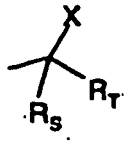

wherein

$R_S$ and $R_T$ are independently selected from the group consisting of hydrogen, alkyl of one to four carbon atoms, phenyl, and substituted phenyl wherein the substituent is selected from the group consisting of alkyl of one to four carbon atoms, alkoxy of one to four carbon atoms, and halogen;

X is selected from the group consisting of alkoxy containing one to four carbon atoms, alkoxyalkyl wherein the alkoxy moiety contains one to four carbon atoms and the alkyl moiety contains one to four carbon atoms, hydroxyalkyl of one to four carbon atoms, haloalkyl of one to four carbon atoms, alkylamido wherein the alkyl group contains one two four carbon atoms, amino, substituted amino wherein the substituent is alkyl or hydroxyalkyl of one to four carbon atoms, azido, chloro, hydroxy, 1-morpholino, 1-pyrrolidino, alkylthio of one to four carbon atoms; and

$R_5$ is selected from the group consisting of hydrogen, straight chain or branched chain alkoxy containing one to four carbon atoms, halogen, and straight chain or branched chain alkyl containing one to four carbon atoms;

and a pharmaceutically acceptable salt of any of the foregoing.

6,7 fused cycloalkylimidazopyridine amine IRM compounds which do not form part of present invention are defined by Formula VI below:

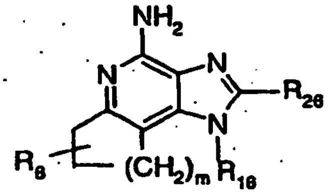

VI

wherein m is 1, 2, or 3;

$R_{16}$ is selected from the group consisting of hydrogen; cyclic alkyl of three, four, or five carbon atoms; straight chain or branched chain alkyl containing one to ten carbon atoms and substituted straight chain or branched chain alkyl containing one to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; fluoro- or chloroalkyl containing from one to ten carbon atoms and one or more fluorine or chlorine atoms; straight chain or branched chain alkenyl containing two to ten carbon atoms and substituted straight chain or branched chain alkenyl containing two to ten carbon atoms, wherein the substituent is

selected from the group consisting of cycloalkyl containing three to six carbon atoms and cycloalkyl containing three to six carbon atoms substituted by straight chain or branched chain alkyl containing one to four carbon atoms; hydroxyalkyl of one to six carbon atoms; alkoxyalkyl wherein the alkoxy moiety contains one to four carbon atoms and the alkyl moiety contains one to six carbon atoms; acyloxyalkyl wherein the acyloxy moiety is alkanoyloxy of two to four carbon atoms or benzoyloxy, and the alkyl moiety contains one to six carbon atoms, with the proviso that any such alkyl, substituted alkyl, alkenyl, substituted alkenyl, hydroxyalkyl, alkoxyalkyl, or acyloxyalkyl group does not have a fully carbon substituted carbon atom bonded directly to the nitrogen atom; benzyl; (phenyl)ethyl; and phenyl; said benzyl, (phenyl)ethyl or phenyl substituent being optionally substituted on the benzene ring by one or two moieties independently selected from the group consisting of alkyl of one to four carbon atoms, alkoxy of one to four carbon atoms, and halogen, with the proviso that when said benzene ring is substituted by two of said moieties, then the moieties together contain no more than six carbon atoms;

and $-CHR_xR_y$

wherein

$R_y$ is hydrogen or a carbon-carbon bond, with the proviso that when $R_y$ is hydrogen $R_x$ is alkoxy of one to four carbon atoms, hydroxyalkoxy of one to four carbon atoms, 1-alkynyl of two to ten carbon atoms, tetrahydropyranyl, alkoxyalkyl wherein the alkoxy moiety contains one to four carbon atoms and the alkyl moiety contains one to four carbon atoms, 2-, 3-, or 4-pyridyl, and with the further proviso that when $R_y$ is a carbon-carbon bond $R_y$ and $R_x$ together form a tetrahydrofuranyl group optionally substituted with one or more substituents independently selected from the group consisting of hydroxy and hydroxyalkyl of one to four carbon atoms,

$R_{26}$ is selected from the group consisting of hydrogen, straight chain or branched chain alkyl containing one to eight carbon atoms, straight chain or branched chain hydroxyalkyl containing one to six carbon atoms morpholinoalkyl, benzyl, (phenyl)ethyl and phenyl, the benzyl, (phenyl)ethyl or phenyl substituent being optionally substituted on the benzene ring by a moiety selected from the group consisting of methyl, methoxy, and halogen; and

$-C(R_S)(R_T)(X)$ wherein $R_S$ and $R_T$ are independently selected from the group consisting of hydrogen, alkyl of one to four carbon atoms, phenyl, and substituted phenyl wherein the substituent is selected from the group consisting of alkyl of one to four carbon atoms, alkoxy of one to four carbon atoms, and halogen;

X is selected from the group consisting of alkoxy containing one to four carbon atoms, alkoxyalkyl wherein the alkoxy moiety contains one to four carbon atoms and the alkyl moiety contains one to four carbon atoms, haloalkyl of one to four carbon atoms, alkylamido wherein the alkyl group contains one to four carbon atoms, amino, substituted amino wherein the substituent is alkyl or hydroxyalkyl of one to four carbon atoms, azido, alkylthio of one to four carbon atoms, and morpholinoalkyl wherein the alkyl moiety contains one to four carbon atoms, and

$R_6$ is selected from the group consisting of hydrogen, fluoro, chloro, straight chain or branched chain alkyl containing one to four carbon atoms, and straight chain or branched chain fluoro- or chloroalkyl containing one to four carbon atoms and at least one fluorine or chlorine atom;

and pharmaceutically acceptable salts thereof.

Imidazopyridine amine IRM compounds which do not form part of present invention are defined by Formula VII below:

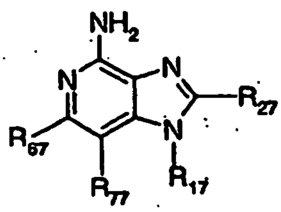

VII

wherein

$R_{17}$ is selected from the group consisting of hydrogen; $-CH_2R_W$ wherein $R_W$ is selected from the group consisting of straight chain, branched chain, or cyclic alkyl containing one to ten carbon atoms, straight chain or branched chain alkenyl containing two to ten carbon atoms, straight chain or branched chain hydroxyalkyl containing one to six carbon atoms, alkoxyalkyl wherein the alkoxy moiety contains one to four carbon atoms and the alkyl moiety contains one to six carbon atoms, and phenylethyl; and $-CH=CR_ZR_Z$ wherein each $R_Z$ is independently straight chain, branched chain, or cyclic alkyl of one to six carbon atoms;

$R_{27}$ is selected from the group consisting of hydrogen, straight chain or branched chain alkyl containing one to eight carbon atoms, straight chain or branched chain hydroxyalkyl containing one to six carbon atoms, alkoxyalkyl wherein

the alkoxy moiety contains one to four carbon atoms and the alkyl moiety contains one to six carbon atoms, benzyl, (phenyl)ethyl and phenyl, the benzyl, (phenyl)ethyl or phenyl substituent being optionally substituted on the benzene ring by a moiety selected from the group consisting of methyl, methoxy, and halogen; and morpholinoalkyl wherein the alkyl moiety contains one to four carbon atoms;

$R_{67}$ and $R_{77}$ are independently selected from the group consisting of hydrogen and alkyl of one to five carbon atoms, with the proviso that $R_{67}$ and $R_{77}$ taken together contain no more than six carbon atoms, and with the further proviso that when $R_{77}$ is hydrogen then $R_{67}$ is other than hydrogen and $R_{27}$ is other than hydrogen or morpholinoalkyl, and with the further proviso that when $R_{67}$ is hydrogen then $R_{77}$ and $R_{27}$ are other than hydrogen;

and pharmaceutically acceptable salts thereof.

1,2-bridged imidazoquinoline amine IRM compounds which do not form part of present invention are defined by Formula VIII below:

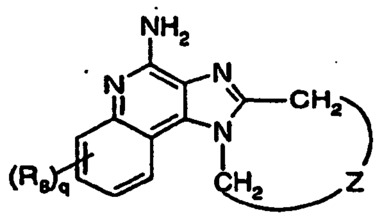

VIII

wherein

Z is selected from the group consisting of:

$-(CH_2)_p-$ wherein p is 1 to 4;

$-(CH_2)_a-C(R_DR_E)(CH_2)_b-$, wherein a and b are integers and a+b is 0 to 3, $R_D$ is hydrogen or alkyl of one to four carbon atoms, and $R_E$ is selected from the group consisting of alkyl of one to four carbon atoms, hydroxy, $-OR_F$ wherein $R_F$ is alkyl of one to four carbon atoms, and $-NR_GR'_G$ wherein $R_G$ and $R'_G$ are independently hydrogen or alkyl of one to four carbon atoms; and

$-(CH_2)_a-(Y)-(CH_2)_b-$ wherein a and b are integers and a+b is 0 to 3, and Y is O, S, or $-NR_J-$ wherein $R_J$ is hydrogen or alkyl of one to four carbon atoms;

and wherein q is 0 or 1 and $R_8$ is selected from the group consisting of alkyl of one to four carbon atoms, alkoxy of one to four carbon atoms, and halogen,

and pharmaceutically acceptable salts thereof.

Thiazolo- and oxazolo- quinolinamine and pyridinamine compounds which do not form part of present invention include compounds of Formula IX:

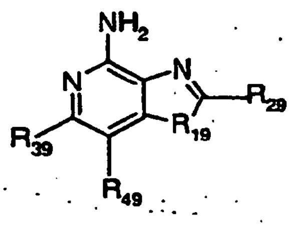

IX

wherein:

$R_{19}$ is selected from the group consisting of oxygen, sulfur and selenium;

$R_{29}$ is selected from the group consisting of

-hydrogen;

-alkyl;

-alkyl-OH;

-haloalkyl;

-alkenyl;

-alkyl-X-alkyl;

-alkyl-X-alkenyl;

-alkenyl-X-alkyl;
-alkenyl-X-alkenyl;
-alkyl-N($R_{59}$)$_2$;
-alkyl-$N_3$;
-alkyl-O-C(O)-N($R_{59}$)$_2$;
-heterocyclyl;
-alkyl-X-heterocyclyl;
-alkenyl-X-heterocyclyl;
-aryl;
-alkyl-X-aryl;
-alkenyl-X-aryl;
-heteroaryl;
-alkyl-X-heteroaryl; and
-alkenyl-X-heteroaryl;
$R_{39}$ and $R_{49}$ are each independently:
-hydrogen;
-X-alkyl;
-halo;
-haloalkyl;
-N($R_{59}$)$_2$;
or when taken together, $R_{39}$ and $R_{49}$ form a fused aromatic, heteroaromatic, cycloalkyl or heterocyclic ring;
X is selected from the group consisting of -O-, -S-, -$NR_{59}$-, -C(O)-, -C(O)O-, -OC(O)-, and a bond; and
each $R_{59}$ is independently H or $C_{1-8}$alkyl;
and pharmaceutically acceptable salts thereof.

Imidazonaphthyridine and tetrahydroimidazonaphthyridine IRM compounds which do not form part of present invention are those of Formulae X and XI below:

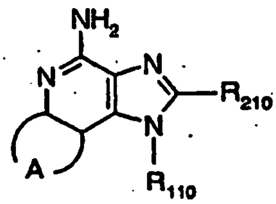

**X**

wherein
A is=N-CR=CR-CR=; =CR-N==CR-CR=; =CR-CR=N-CR=; or =CR-CR=CR-N=;
$R_{110}$ is selected from the group consisting of:

- hydrogen;

-$C_{1-20}$ alkyl or $C_{2-20}$ alkenyl that is unsubstituted or substituted by one or more substituents selected from the group consisting of:
-aryl;
-heteroaryl;
-heterocyclyl;
-O-$C_{1-20}$ alkyl,
-O-($C_{1-20}$alkyl)$_{0-1}$-aryl;
-O-($C_{1-20}$alkyl)$_{0-1}$-heteroaryl;
-O-($C_{1-20}$alkyl)$_{0-1}$-heterocyclyl;
-$C_{1-20}$ alkoxycarbonyl;
-S(O)$_{0-2}$-$C_{1-20}$ alkyl;
-S(O)$_{0-2}$-($C_{1-20}$ alkyl)$_{0-1}$-aryl;
-S(O)$_{0-2}$-($C_{1-20}$ alkyl)$_{0-1}$-heteroaryl;

-$S(O)_{0-2}$-($C_{1-20}$ alkyl)$_{0-1}$-heterocyclyl;
-$N(R_{310})_2$;
-$N_3$;
oxo;
-halogen;
-$NO_2$;
-OH; and
-SH; and
-$C_{1-20}$ alkyl-$NR_{310}$-Q-X-$R_{410}$ or -$C_{2-20}$ alkenyl-$NR_{310}$-Q-X-$R_{410}$ wherein **Q** is -CO- or-$SO_2$-; **X** is a bond, -O- or -$NR_{310}$- and $R_{410}$ is aryl; heteroaryl; heterocyclyl; or -$C_{1-20}$ alkyl or $C_{2-20}$ alkenyl that is unsubstituted or substituted by one or more substituents selected from the group consisting of:
-aryl;
-heteroaryl;
-heterocyclyl;
-O-$C_{1-20}$ alkyl,
-O-($C_{1-20}$alkyl)$_{0-1}$-aryl;
-O-($C_{1-20}$alkyl)$_{0-1}$-heteroaryl;
-O-($C_{1-20}$alkyl)$_{0-1}$-heterocyclyl;
-$C_{1-20}$ alkoxycarbonyl;
-$S(O)_{0-2}$-$C_{1-20}$alkyl;
-$S(O)_{0-2}$-($C_{1-20}$alkyl)$_{0-1}$-aryl;
-$S(O)_{0-2}$-($C_{1-20}$alkyl)$_{0-1}$-heteroaryl;
-$S(O)_{0-2}$-($C_{1-20}$alkyl)$_{0-1}$-heterocyclyl;
-$N(R_{310})_2$;
-$NR_{310}$-CO-O-$C_{1-20}$alkyl;
-$N_3$;
oxo;
-halogen;
-$NO_2$;
-OH; and
-SH; or $R_{410}$ is

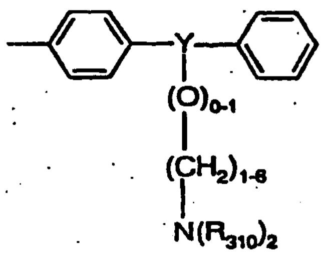

wherein **Y** is N- or -CR-;
$\mathbf{R_{210}}$ is selected from the group consisting of:
-hydrogen;
-$C_{1-10}$ alkyl;
-$C_{2-10}$ alkenyl;
-aryl;
-$C_{1-10}$ alkyl-O-$C_{1-10}$-alkyl;
-$C_{1-10}$ alkyl-O-$C_{2-10}$ alkenyl; and
-$C_{1-10}$ alkyl or $C_{2-10}$ alkenyl substituted by one or more substituents selected from the group consisting of:
-OH;
-halogen;
-$N(R_{310})_2$;
-CO-$N(R_{310})_2$;
-CO-$C_{1-10}$ alkyl;
-$N_3$;
-aryl;

-heteroaryl;
-heterocyclyl;
-CO-aryl; and
-CO-heteroaryl;
each $\mathbf{R_{310}}$ is independently selected from the group consisting of hydrogen and $C_{1-10}$ alkyl; and
each **R** is independently selected from the group consisting of hydrogen, $C_{1-10}$ alkyl, $C_{1-10}$ alkoxy, halogen and trifluoromethyl,
and pharmaceutically acceptable salts thereof.

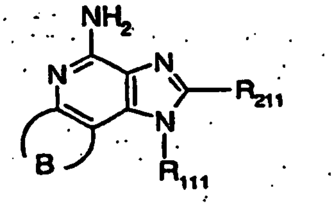

XI

wherein
**B** is $-NR-C(R)_2-C(R)_2-C(R)_2-$; $-C(R)_2-NR-C(R)_2-C(R)_2-$;
$-C(R)_2-C(R)_2-NR-C(R)_2-$ or $-C(R)_2-C(R)_2-C(R)_2-NR-$;
$\mathbf{R_{111}}$ is selected from the group consisting of:
- hydrogen;
-$C_{1-20}$ alkyl or $C_{2-20}$ alkenyl that is unsubstituted or substituted by one or more substituents selected from the group consisting of:
-aryl;
-heteroaryl;
-heterocyclyl;
-O-$C_{1-20}$alkyl;
-O-($C_{1-20}$alkyl)$_{0-1}$-aryl;
-O-($C_{1-20}$alkyl)$_{0-1}$-heteroaryl;
-O-($C_{1-2}$alkyl)$_{0-1}$-heterocyclyl;
-$C_{1-20}$ alkoxycarbonyl;
-S(O)$_{0-2}$-$C_{1-20}$alkyl;
-S(O)$_{0-2}$-($C_{1-20}$alkyl)$_{0-1}$-aryl;
-S(O)$_{0-2}$-($C_{1-20}$alkyl)$_{0-1}$-heteroaryl;
-S(O)$_{0-2}$-($C_{1-20}$ alkyl)$_{0-1}$-heterocyclyl;
-N($R_{311}$)$_2$;
-$N_3$;
oxo;
-halogen;
-$NO_2$;
-OH; and
-SH; and
-$C_{1-20}$ alkyl-$NR_{311}$-Q-X-$R_{411}$ or-$C_{2-20}$ alkenyl-$NR_{311}$-Q-X-$R_{411}$ wherein **Q** is -CO- or -$SO_2$-; **X** is a bond, -O- or -$NR_{311}$- and $\mathbf{R_{411}}$ is aryl; heteroaryl; heterocyclyl; or -$C_{1-20}$ alkyl or $C_{2-20}$ alkenyl that is unsubstituted or substituted by one or more substituents selected from the group consisting of:
-aryl;
-heteroaryl;
-heterocyclyl;
-O-$C_{1-20}$alkyl,
-O-($C_{1-20}$alkyl)$_{0-1}$-aryl;
-O-($C_{1-20}$alkyl)$_{0-1}$-heteroaryl;
-O-($C_{1-20}$alkyl)$_{0-1}$-heterocyclyl;
-$C_{1-20}$alkoxycarbonyl;
-S(O)$_{0-2}$-$C_{1-20}$ alkyl;
-S(O)$_{0-2}$ -($C_{1-20}$ alkyl)$_{0-1}$-aryl;

$-S(O)_{0-2}-(C_{1-20}$ alkyl$)_{0-1}$-heteroaryl;
$-S(O)_{0-2}-(C_{1-20}$ alkyl$)_{0-1}$-heterocyclyl;
$-N(R_{311})_2$;
$-NR_{311}-CO-O-C_{1-20}$alkyl;
$-N_3$;
oxo;
-halogen;
$-NO_2$;
-OH; and
-SH; or $R_{411}$ is

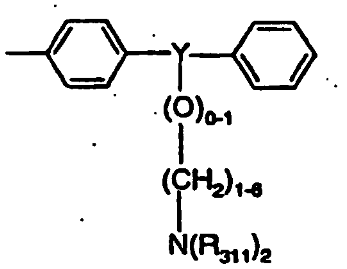

wherein **Y** is -N- or -CR-;
$\mathbf{R_{211}}$ is selected from the group consisting of:
-hydrogen;
$-C_{1-10}$ alkyl;
$-C_{2-10}$ alkenyl;
-aryl
$-C_{1-10}$ aryl-O-$C_{1-10}$-alkyl;
$-C_{1-10}$ alkyl-O-$C_{2-10}$ alkenyl; and
$-C_{1-10}$ alkyl or $C_{2-10}$ alkenyl substituted by one or more substituents selected from the group consisting of:
-OH;
-halogen;
$-N(R_{311})_2$;
$-CO-N(R_{311})_2$;
$-CO-C_{1-10}$ alkyl;
$-N_3$;
-aryl;
-heteroaryl;
-heterocyclyl;
-CO-aryl; and
-CO-heteroaryl;
each $\mathbf{R_{311}}$ is independently selected from the group consisting of hydrogen and $C_{1-10}$ alkyl; and
each **R** is independently selected from the group consisting of hydrogen, $C_{1-10}$ alkyl, $C_{1-10}$ alkoxy, halogen and trifluoromethyl,
and pharmaceutically acceptable salts thereof. Compounds of formula XI do not form part of present invention
1*H*-imidazo[4,5-*c*]quinolin-4-amines and tetrahydro-1*H*-imidazo[4,5-*c*]quinolin-4-amines include compounds defined by Formulas XII, XIII and XIV below which do not form part of present invention

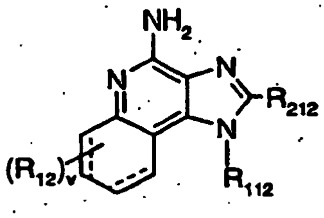

XII

wherein

$R_{112}$ is -alkyl-$NR_{312}$-CO-$R_{412}$ or -alkenyl-$NR_{312}$-CO- $R_{412}$ wherein $\mathbf{R_{412}}$ is aryl, heteroaryl, alkyl or alkenyl, each of which may be unsubstituted or substituted by one or more substituents selected from the group consisting of:

-alkyl;
-alkenyl;
-alkynyl;
-(alkyl)$_{0-1}$-aryl;
-(alkyl)$_{0-1}$-(substituted aryl);
-(alkyl)$_{0-1}$-heteroaryl;
-(alkyl)$_{0-1}$-(substituted heteroaryl);
-O-alkyl;
-O-(alkyl)$_{0-1}$-aryl;
-O-(alkyl)$_{0-1}$ -(substituted aryl);
-O-(alkyl)$_{0-1}$-heteroaryl;
-O-(alkyl)$_{0-1}$-(substituted heteroaryl);
-CO-aryl;
-CO-(substituted aryl);
-CO-heteroaryl;
-CO-(substituted heteroaryl);
-COOH;
-CO-O-alkyl;
-CO-alkyl;
-S(O)$_{0-2}$-alkyl;
-S(O)$_{0-2}$-(alkyl)$_{0-1}$-aryl;
-S(O)$_{0-2}$-(alkyl)$_{0-1}$-(substituted aryl);
-S(O)$_{0-2}$-(alkyl)$_{0-1}$-heteroaryl;
-S(O)$_{0-2}$-(alkyl)$_{0-1}$-(substituted heteroaryl);
-P(O)($OR_{312}$)$_2$;
-$NR_{312}$-CO-O-alkyl;
-$N_3$;
-halogen;
-$NO_2$;
-CN;
-haloalkyl;
-O-haloalkyl;
-CO-haloalkyl;
-OH;
-SH; and in the case of alkyl, alkenyl, or heterocyclyl, oxo;

or $\mathbf{R_{412}}$ is

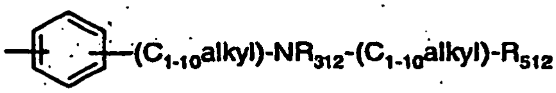

wherein $\mathbf{R_{512}}$ is an aryl, (substituted aryl), heteroaryl, (substituted heteroaryl), heterocyclyl or (substituted heterocyclyl) group;

$\mathbf{R_{212}}$ is selected from the group consisting of:

-hydrogen;
-alkyl;
-alkenyl;
-aryl;
-(substituted aryl);
-heteroaryl;
-(substituted heteroaryl);
-heterocyclyl;
-(substitute heterocyclyl);
-alkyl-O-alkyl;

-alkyl-O-alkenyl; and
-alkyl or alkenyl substituted by one or more substituents selected from the group consisting of:
-OH;
-halogen;
$-N(R_{312})_2$;
$-CO-N(R_{312})_2$;
$-CO-C_{1-10}$ alkyl;
$-CO-O-C_{1-10}$ alkyl;
$-N_3$;
-aryl;
-(substituted aryl);
-heteroaryl;
-(substituted heteroaryl);
-heterocyclyl;
-(substituted heterocyclyl);
-CO-aryl; and
-CO-heteroaryl;
each $R_{312}$ is independently selected from the group consisting of hydrogen; $C_{1-10}$ alkyl-heteroaryl; $C_{1-10}$ alkyl-(substituted heteroaryl); $C_{1-10}$ alkyl-aryl; $C_{1-10}$ akyl-(substituted aryl) and $C_{1-10}$ alkyl;
v is 0 to 4;
and each $R_{12}$ present is independently selected from the group consisting of $C_{1-10}$ alkyl, $C_{1-10}$ alkoxy, halogen and trifluoromethyl;

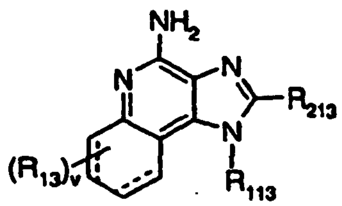

XIII

wherein
$R_{113}$ is $-alkyl-NR_{313}-SO_2-X-R_{413}$ or $-alkenyl-NR_{313}-SO_2-X-R_{413}$;
X is a bond or$-NR_{513}-$;
$R_{413}$ is acryl, heteroaryl, heterocyclyl, alkyl or alkenyl, each of which may be unsubstituted or substituted by one or more substituents selected from the group consisting of:
-alkyl;
-alkenyl;
-aryl;
-heteroaryl;
-heterocyclyl;
-substituted cycloalkyl;
-substituted aryl;
-substituted heteroaryl;
-substituted heterocyclyl;
-O-alkyl;
$-O-(alkyl)_{0-1}$-aryl;
$-O-(alkyl)_{0-1}$-substituted aryl;
$-O-(alkyl)_{0-1}$-heteroaryl;
$-O-(alkyl)_{0-1}$-substituted heteroaryl;
$-O-(alkyl)_{0-1}$-heterocyclyl;
$-O-(alkyl)_{0-1}$-substituted heterocyclyl;
-COOH;
-CO-O-alkyl;
-CO-alkyl;

$-S(O)_{0-2}$-alkyl;
$-S(O)_{0-2}-(alkyl)_{0-1}$-aryl;
$-S(O)_{0-2}-(alkyl)_{0-1}$-substituted aryl;
$-S(O)_{0-2}-(alkyl)_{0-1}$-heteroaryl;
$-S(O)_{0-2}-(alkyl)_{0-1}$-substituted heteroaryl;
$-S(O)_{0-2}-(alkyl)_{0-1}$-heterocyclyl;
$-S(O)_{0-2}-(alkyl)_{0-1}$-substituted heterocyclyl;
$-(alkyl)_{0-1}-NR_{313}R_{313}$;
$-(alkyl)_{0-1}-NR_{313}$-CO-O-alkyl;
$-(alkyl)_{0-1}-NR_{313}$-CO-alkyl;
$-(alkyl)_{0-1}-NR_{313}$-CO-aryl;
$-(alkyl)_{0-1}NR_{313}$-CO-substituted aryl;
$-(alkyl)_{0-1}-NR_{313}$-CO-heteroaryl;
$-(alkyl)_{0-1}-NR_{313}$-CO-substituted heteroaryl;
$-N_3$;
-halogen;
-haloalkyl;
-haloalkoxy;
-CO-haloalkyl;
-CO-haloalkoxy;
$-NO_2$;
-CN;
-OH;
-SH; and in the case of alkyl, alkenyl, or heterocyclyl, oxo;
$R_{213}$ is selected from the group consisting of:
-hydrogen;
-alkyl;
-alkenyl;
-aryl;
-substituted aryl;
-heteroaryl;
-substituted heteroaryl;
-alkyl-O-alkyl;
- alkyl-O- alkenyl; and
-alkyl or alkenyl substituted by one or more substituents selected from the group consisting of:
-OH;
-halogen;
$-N(R_{313})_2$;
$-CO-N(R_{313})_2$;
$-CO-C_{1-10}$ alkyl;
$-CO-O-_{C1-10}$ alkyl;
$-N_3$;
-aryl;
-substituted aryl;
-heteroaryl;
-substituted heteroaryl;
-heterocyclyl;
-substituted heterocyclyl;
-CO-aryl;
-CO-(substituted aryl);
-CO-heteroaryl; and
-CO-(substituted heteroaryl);
each $R_{313}$ is independently selected from the group consisting of hydrogen and $C_{1-10}$ alkyl;
$R_{513}$ is selected from the group consisting of hydrogen and $C_{1-10}$ alkyl, or $R_{413}$ and $R_{513}$ can combine to form a 3 to 7 membered heterocyclic or substituted heterocyclic ring;
v is 0 to 4 and each $R_{13}$ present is independently selected from the group consisting of $C_{1-10}$ alkyl, $C_{1-10}$ alkoxy, halogen and trifluoromethyl;

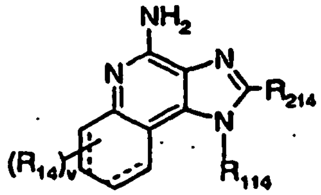

XIV

wherein
$R_{114}$ is -alkyl-$NR_{314}$-CY-$NR_{514}$-X-$R_{414}$ or
-alkenyl-$NR_{314}$-CY-$NR_{514}$-X-$R_{414}$
wherein
Y is =O or =S;
X is a bond, -CO- or -$SO_2$-;
$R_{414}$ is aryl, heteroaryl, heterocyclyl, alkyl or alkenyl, each of which may be unsubstituted or substituted by one or more substituents selected from the group consisting; of:
-alkyl;
-alkenyl,
-aryl;
-heteroaryl;
-heterocyclyl;
-substituted aryl;
-substituted heteroaryl;
-substituted heterocyclyl;
-O-alkyl;
-O-$(alkyl)_{0-1}$-aryl;
-O-$(alkyl)_{0-1}$-substituted aryl;
-O-$(alkyl)_{0-1}$-heteroaryl;
-O-$(alkyl)_{0-1}$-substituted heteroaryl;
-O-$(alkyl)_{0-1}$-heterocyclyl;
-O-$(alkyl)_{0-1}$-substituted heterocyclyl;
-COOH;
-CO-O-alkyl;
-CO-alkyl;
-$S(O)_{0-2}$-alkyl;
-$S(O)_{0-2}$-$(alkyl)_{0-1}$-aryl;
-$S(O)_{0-2}$-$(alkyl)_{0-1}$-substituted aryl;
-$S(O)_{0-2}$-$(alkyl)_{0-1}$-heteroaryl;
-$S(O)_{0-2}$-$(alkyl)_{0-1}$-substituted heteroaryl;
$S(O)_{0-2}$-$(alkyl)_{0-1}$-heterocyclyl;
-$S(O)_{0-2}$-$(alkyl)_{0-1}$-substituted heterocyclyl;
-$(alkyl)_{0-1}$-$NR_{314}R_{314}$;
-$(alkyl)_{0-1}$-$NR_{314}$-CO-O-alkyl;
-$(alkyl)_{0-1}$-$NR_{314}$-CO-alkyl;
-$(alkyl)_{0-1}$-$NR_{314}$-CO-aryl;
-$(alkyl)_{0-1}$-$NR_{314}$-CO-substituted aryl;
-$(alkyl)_{0-1}NR_{314}$-CO-heteroaryl;
-$(alkyl)_{0-1}$-$NR_{314}$-CO-substituted heteroaryl;
-$N_3$;
-halogen;
-haloalkyl;
-haloalkoxy;
-CO-haloalkoxy;
-$NO_2$;
-CN;

-OH;
-SH; and, in the case of alkyl, alkenyl or heterocyclyl, oxy; with the proviso that when X is a bond $R_{414}$ can additionally be hydrogen;
$R_{214}$ is selected from the group consisting of:
-hydrogen;
-alkyl;
-alkenyl;
-aryl;
-substituted aryl;
-heteroaryl;
-substituted heteroaryl;
-alkyl-O-alkyl;
-alkyl-O- alkenyl; and
alkyl or alkenyl substituted by one or more substituents selected from the group consisting of:
-OH;
-halogen;
$-N(R_{314})_2$;
$-CO-N(R_{314})_2$;
-CO-$C_{1-10}$ alkyl;
-CO-O-$C_{1-10}$ alkyl;
$-N_3$;
-aryl;
-substituted aryl;
-heteroaryl;
-substituted heteroaryl;
-heterocyclyl;
-substituted heterocyclyl;
-CO-aryl;
-CO-(substituted aryl);
-CO-heteroaryl; and
-CO-(substituted heteroaryl);
each $R_{314}$ is independently selected from the group consisting of hydrogen and $C_{1-10}$ alkyl;
$R_{514}$ is selected from the group consisting of hydrogen and $C_{1-10}$ alkyl, or $R_{414}$ and $R_{514}$ can combine to form a 3 to 7 membered heterocyclic or substituted heterocyclic ring;
v is 0 to 4 and each $R_{14}$ present is independently selected from the group consisting of $C_{1-10}$ alkyl, $C_{1-10}$ alkoxy, halogen and trifluoromethyl, and a pharmaceutically acceptable salts thereof.

Known IRM compounds also include the purine derivatives, small heterocyclic compounds, amide derivatives, and oligonucleotide sequences described above.

## Examples

The following examples have been selected merely to further illustrate features, advantages, and other details of the invention. All the compounds not included in claim 1 do not form part of present invention, and are to be considered as reference examples.

### Compounds

The compounds used in the following Examples and citations for methods for synthesizing each compound are provided in Table 1.

**Table 1**

| Compound | Chemical Name | Citation |
|---|---|---|
| Imiquimod | 1-(2-methylpropyl)1*H*-imidazo[4,5-*c*]quinolin-4-amine | U.S. 4,689,338 Example 99 |
| Resiquimod | 4-amino-2-ethoxymethyl-α,α-dimethyl-1*H*-imidazo[4,5-c] quinoline-1-ethanol | U.S. 5,389,640 Example 99 |

(continued)

| Compound | Chemical Name | Citation |
|---|---|---|
| IRM 1 | 4-amino-α,α,2-trimethyl-1*H*-imidazo[4,5-*c*]quinoline-1-ethanol hydrochloride | U.S. 5,266,575 Example C1* |
| IRM 2 | 2-propylthiazolo[4,5-*c*]quinolin-4-amine | U.S. 6,110,929 Example 12 |
| IRM 3 | N-[4-(4-amino-2-butyl-1*H*-imidazo[4,5-*c*][1,5]naphthyridin-1-yl) butyl]-N'-cyclohexylurea | U.S. 6,194,425 Example 48 |
| IRM 4 | 1-{2-[3-(3-pyridyl)propoxy]ethyl}-1*H*-imidazo[4,5-*c*]quinolin-4-amine | WO 02/46193 Example 33 |
| IRM 5 | 2-butyl-1-(2-methylpropyl)-1*H*-imidazo[4,5-*c*][1,8]naphthyridin-4-amine | U.S. 6,194,425 Example 12 |
| IRM 6 | 2-butyl-1-(2-methylpropyl)-1*H*-imidazo[4,5-*c*][1,7]naphthyridin-4-amine | U.S. 6,194,425 Example 27 |
| IRM 7 | 2-butyl-1-(2-methylpropyl)-1*H*-imidazo[4,5-*c*][1,5]naphthyridin-4-amine | U.S. 6,194,425 Example 39 |
| IRM 8 | 2-butyl-6,7,8,9-tetrahydro-1-(2-methylpropyl)-1*H*-imidazo[4,5-*c*] [1,5]naphthyridin-4-amine | U.S. 6,194,425 Example 40 ' |
| IRM 9 | 4-amino-2-ethoxymethyl-α,α-dimethyl-6,7,8,9-tetrahydro-1*H*-imidazo[4,5-*c*]quinoline-1-ethanol | U.S. 5,352,784 Example 91 |
| IRM 10 | 1-[R(+)-1-phenylethyl]-1*H*-imidazo[4,5-*c*]quinolin-4-amine | U.S. 4,689,338 Example 185** |
| IRM 11 | 2-butyl[1,3]thiazolo[4,5-*c*][1,5]naphthyridin-4-amine | U.S. 6,110,929 Example 58 |
| IRM 12 | N-[4-(4-amino-2-butyl-1*H*-imidazo[4,5-*c*]quinolin-1-yl)butyl] methanesulfonamide | U.S. 6,331,539 Example 6 |
| IRM 13 | 8,9,10,11-tetrahydropyrido[1',2':1,2]imidazo[4,5-*c*]quinolin-6-amine | U.S. 5,482,936 Example 1 |
| IRM 14 | $N^3$-{4-[4-amino-2-(2-methoxyethyl)-1*H*-imidazo[4,5-*c*]quinolin-1-yl]butyl}-6-(1*H*-1-pyrrolyl)nicotinamide | U.S. 6,451,810 Example 60 |
| IRM 15 | N-[2-4-amino-2-butyl-1*H*-imidazo[4,5-*c*]quinolin-1-yl)ethyl] methanesulfonamide | U.S. 6,331,539 Example 34*** |
| IRM 16 | N-{4-[4-amino-2-(2-methoxyethyl)-1*H*-imidazo[4,5-*c*]quinolin-1-yl] butyl}morpholine-4-carboxamide | WO 00/76518 Example 121*** |
| IRM 17 | N-[4-(4-amino-2-butyl-6,7-dimethyl-1*H*-imidazo[4,5-*c*]pyridin-1-yl) butyl]methanesulfonamide | WO 02/46194 Example 2 |
| IRM 18 | 2-ethyl-1-[5-(methylsulfonyl)pentyl]-1*H*-imidazo[4,5-*c*]quinolin-4-amine | WO 02/46192 Example 13 |
| *Example C1 is the free base. The hydrochloride salt is prepared using conventional methods.<br>**Example 185 is the racemic mixture. The chiral compound is prepared using (R)-(+)-α-methylbenzylamine (available from Aldrich. Milwaukee, WI, USA).<br>***Examples 34 and 121 are trifluoroacetate salts. The salt is converted to the free base using conventional methods. | | |

## Cells

**HEK293 cells -** immortalized human embryonic kidney cells, available from American Type Culture Collection, Manassas, VA, ATCC No. CRL-1573.

**RAW 264.7 cells -** mouse macrophage cells, available from American Type Tissue Collection, Manassas, VA, ATCC No. TIB-71.

**THP-1 cells** - human monocyte cells derived from acute monocytic leukemia tissue; available from American Type Culture Collection, Manassas, VA, ATCC No. TIB-202.

**Cell Culture Media**

Complete RPMI was prepared by mixing RPMI 1640 with 25mM HEPES, 1 mM sodium pyruvate, 0.1 mM non-essential amino acids, and 1 mM L-glutamine (Celox Laboratories, Inc., Minneapolis, MN) supplemented with 10% heat inactivated fetal calf serum (FCS) (Hyclone Laboratories, Inc., Logan, UT) and 1% penicillin/streptomycin (Sigma Chemical Co., St. Louis, MO). For the transfection of dominant negative constructs into THP-1 cells, cRPMI was modified by the addition of 3.5 g/L glucose and 5 x $10^{-5}$ M2-mercaptoethanol (tRPMI). For the transfection of dominant negative constructs into RAW 264.7 cells, cRPMI was modified by the addition of 5 x $10^{-5}$ M 2-mercaptoethanol (rRPMI).

**Example 1- The Effect of Dominant Negative TLR 6 and TLR 7**

A murine TLR6 dominant negative construct was generated by PCR mutation during amplification from RAW 264.7 cell cDNA. The 5' and 3' regions flanking a codon encoding proline 691 were amplified with primers (5' sense: SEQ ID NO 1; 5' antisense: SEQ ID NO 2; 3' sense: SEQ ID NO 3; 3' antisense: SEQ ID NO 4) that changed the codon for proline 691 to a codon encoding histidine while introducing a unique Apa LI restriction enzyme site at the position of the mutation. The 5' and 3' sections of the TLR6 were amplified by Pfu Turbo DNA polymerase kit (Stratagene, La Jolla, CA). The PCR sections were inserted into pCR-Blunt II-TOPO for sequence verification. The two sections were joined together when subcloned into pIRES (Clontech, Palo Alto, CA) for expression in mammalian cells.

The human TLR6 dominant negative construct was generated from human PBMC cDNA using the same strategy as the murine TLR6 dominant negative. The proline to histidine mutation for human TLR6 was introduced at amino acid 680 along with an Apa LI restriction enzyme site (5' sense: SEQ ID NO 5; 5' antisense: SEQ ID NO 6; 3' sense: SEQ ID NO 7; 3' antisense: SEQ ID NO 8).

The human TLR7 dominant negative construct was generated in a manner similar to that used to generate the human TLR6 dominant negative construct. The proline to histidine mutation for human was introduced at amino acid 932 along with a Bam HI restriction enzyme site (5' sense: SEQ ID NO 9; 5' antisense: SEQ ID NO 10; 3' sense: SEQ ID NO 11; 3' antisense: SEQ ID NO 12).

The amplified 5' and 3' sections of each human dominant negative TLR was inserted into pCR-Blunt II-TOPO for sequence verification. The 5' and 3' sections were joined together when subcloned into pIRES (Clontech, Palo Alto, CA) for expression in mammalian cells.

THP-1 cells (maintained at cell number less than 1 x $10^6$ cells/ ml) were co-transfected with the plamid vector containing either the TLR6DN or TLR7DN construct and with a murine $H_2K^k$ plasmid (Miltenyi Biotec Inc., Auburn, CA) in a 4:1 ratio of TLR plasmid to $H_2K^k$ plasmid. Transfection of THP-1 cells was carried out using the transfection reagent FuGENE 6 (Roche Diagnostics Corp., Indianapolis, IN) according to the manufacturer's specifications. At 18 hours post-transfection, transfected cells were selected on the basis of murine $H_2K^k$ (Miltenyi Biotec Inc., Auburn, CA) according to the manufacturer's specifications.

RAW 264.7 cells were co-transfected with a truncated human CD4 for RAW 264.7 cells in a 4:1 ratio of TLR plasmid to CD4 plasmid. Transfection of RAW 264.7 cells was carried out using the transfection reagent DoTaP (Roche Diagnostics Corp., Indianapolis, IN) according to the manufacturer's specifications. At 18 hours post-transfection, transfected cells were selected on the basis of CD4 expression (Miltenyi Biotec Inc., Auburn, CA) for the RAW 264.7 cells according to the manufacturer's specifications.

After selection, cells were resuspended in tRPMI at a concentration of $10^6$ cells/ml. 100 $\mu$l of cells ($10^5$ cells) were then added to individual wells of a 96 well U-bottom plate (BD Biosciences Discovery Labware, Bedford, MA). The IRM compound was diluted to 6$\mu$M, LPS (Sigma Chemical Co., St. Louis, Mo.) diluted to 200 ng/ml; and zymosan (Sigma Chemical Co., St. Louis, Mo.) was diluted to 6 x $10^5$ particles/ml. After the addition of the compound solution, cells were incubated for 18 hours at 37° C in an atmosphere of 5% $CO_2$/95% air. Supernatants were collected and frozen at -20° C for cytokine analysis.

TNF-$\alpha$ levels were measured with a commercial Human TNF-$\alpha$ ELISA kit (Biosource International, Inc., Camarillo, CA) according to the manufacturer's specifications. Results are presented in % inhibition over vector control.

The data in Table 1 represent results of THP-1 cells transfected with either TLR6DN or TLR7DN, stimulated for 18 hours with 3 $\mu$M resiquimod, 100 ng LPS, or 3x $10^5$ particles of zymosan. Results are presented in % inhibition relative to vector control. Data shown are representative of six independent experiments.

**Table 2 - TNF-$\alpha$ Production by THP-1 Cells Transfected with Either TLR6DN or TLR7DN**

| | TLR6DN | | TLR7DN | |
|---|---|---|---|---|
| Stimulus | % inhibition | SEM | % inhibition | SEM |
| LPS 100 ng/ml | 2.5 | 5.4 | 13.2 | 6.1 |

(continued)

| Stimulus | TLR6DN % inhibition | TLR6DN SEM | TLR7DN % inhibition | TLR7DN SEM |
|---|---|---|---|---|
| Zymosan 3 X$10^5$ particles/ml | 58.2 | 4.2 | 6.9 | 3.2 |
| Resiquimod 3μM | 70.1 | 1.3 | 55.3 | 2.4 |

**Table 3 - TNF-α Production by RAW 264.7 Cells Transfected with TLR6DN**

| Stimulus | % inhibition | SEM |
|---|---|---|
| LPS 100 ng/ml | 17.6 | 1.2 |
| Zymosan 3X$10^5$ particles/ml | 80.7 | 3.9 |
| Resiquimod 3μM | 70.9 | 3.6 |

**Example 2 Antibody Blocking of IRM-Mediated Cell Stimulation**

Rabbit polyclonal antibodies were generated by Quality Controlled Biochemicals, Inc., (Hopkinton, MA). Antibody specificity was verified by flow cytometry and western blotting.

Peripheral blood mononuclear cells (PBMCs) were isolated with the Histopaque HybriMax -1077 density gradient (Sigma Chemical Co., St. Louis, MO) from healthy human volunteers after obtaining informed consent.

PBMC were resuspended in cRPMI at a concentration of $10^6$ cells/ml. 100 μl of cells ($10^5$ cells) were then added to individual wells of a 96 well U-bottom plate (BD Biosciences Discovery Labware, Bedford, MA). Solutions containing cRPMI with 40μ g/ml of the affinity purified anti-TLR6 polyclonal antibody were prepared. 50 μl of the antibody solution was added to cells and incubated for 30 minutes. The IRM compounds were diluted to 12 μM; LPS (Sigma Chemical Co., St. Louis, Mo.) was diluted to 400 ng/ml; zymosan (Sigma Chemical Co., St. Louis, Mo.) was diluted to 12 x $10^5$ particles/ml; and peptidoglycan (Sigma Chemical Co., St. Louis, Mo.) was diluted to 40 μg/ml in cRPMI. 50 μl of the compound solution was added to cells so that the final concentration of antibody was 10 μg/ml, the final concentration of resiquimod was 3 μM, LPS was 100 ng/ml, and peptidoglycan was 10 μg/ml. Cells were incubated for 18 hours at 37° C in an atmosphere of 5% $CO_2$/95% air. Supernatants were collected and frozen at -20° C for cytokine analysis. The data are presented as % inhibition relative to control.

$$\% \text{ inhibition} = 100 \times \frac{(\text{control value} - \text{treated value})}{\text{control value}}$$

The IRM compounds used in this section were synthesized at 3M, St. Paul, MN. The syntheses of these compounds are described in U.S. Patent Nos. 5,389,640: Example 99 (resiquimod); 4,689,338: Example 99 (imiquimod); 5,266,575: Example C1 (Compound 1); 6,194,425: Example 48 (Compound 3); 6,110,929: Example 12 (Compound 2); 6,194,425: Example 12 (Compound 5), Example 27 (Compound 6), Example 39 (compound 7), and Example 40 (Compound 8).

The data in Table 3 represent results of TLR6 neutralizing antibody studies in human PBMC. PBMC were stimulated for 18 hrs with 100 ng/ml LPS, 10 μg/ml peptidoglycan, zymosan particles, or the indicated concentration of IRM compound. Results are presented in % inhibition relative to media control. Data shown are representative of six independent experiments.

**Table 4 - Anti-TLR6 Antibody Inhibition of TNF-α Production by Human PBMC Cells**

| Stimulus | % inhibition relative to control (no Ab) | SEM |
|---|---|---|
| 100 ng/ml LPS | -9.4 | 3.1 |
| 10μ g/ml Peptidoglycan | 50.0 | 7.2 |
| Zymosan 3x$10^5$ particles/ml | 66.1 | 1.8 |
| 3 μM Resiquimod | 88.4 | 4.4 |
| 3μM IRM 1 | 70.2 | 3.7 |
| 3μM IRM 3 | 65.0 | 12.1 |
| 3 μM IRM 2 | 81.0 | 9.3 |

(continued)

| Stimulus | % inhibition relative to control (no Ab) | SEM |
|---|---|---|
| 0.12 μM IRM 4 | 76.7 | 2.4 |
| 3 μM IRM 5 | 84.2 | 8.7 |
| 1 μM IRM 6 | 90.3 | 1.8 |
| 0.37 μM IRM 7 | 78.2 | 8.4 |
| 1μM IRM 8 | 64.7 | 1 |

**Example 3 - Overexpression of Wild-Type TLR 6 or TLR 7**

The murine TLR wild-type vectors were generated by PCR amplification from RAW 264.7 cell cDNA with TLR6 specific primers (sense primer: SEQ ID NO. 13; antisense primer: SEQ ID NO 14) or TLR7 specific primers (sense primer: SEQ ID NO 15; antisense primer: SEQ ID NO 16) by Pfu Turbo DNA polymerase kit (Stratagene, La Jolla, CA). The PCR products were inserted into pCR-Blunt II-TOPO for sequence verification and then subcloned into pIRES (BD Biosciences Clontech, Palo Alto, CA) for expression in mammalian cells.

THP-1 cells or RAW 264.7 cells were cultured and transfected with the wild type TLR 6 or wild type TLR 7 plasmids described above. The transfections were performed as in Example 1 with a 4:1 ratio of wild-type TLR to H2K plasmid (THP-1 cells) or CD4 (RAW 264.7 cells).

RAW 264.7 cells were stimulated with various concentrations of resiquimod and analyzed as described in Example 1. Results are provided in Table 4 and are expressed as fold increase in TNF-α production as compared to control transfected RAW 264.7 cells.

**Table 5 - IRM-Stimutated TNF-α Production by RAW 264.7 Cells Overexpressing TLR6 or TLR7**

| Resiquimod (μM) | Fold increase in TNF-α production over control | |
|---|---|---|
| | TLR 6 | TLR7 |
| | - | - |
| 0.0004 | 9.6 | 14.8 |
| 0.001 | 8.0 | 8.9 |
| 0.004 | 9.2 | 5.8 |
| 0.012 | 3.5 | 3.8 |
| 0.037 | 3.9 | 3.5 |
| 1 | 1.4 | 1.3 |
| 3 | 1.7 | 1.0 |
| 10 | 1.8 | 1.5 |

**Example 4 - Overexpression of TLR7 in HEK293 cells**

HEK 293 cells were cultured in Minimum Essential Medium (MEM) with 2 mM L-glutamine and Earle's Balanced Salt Solution (Invitrogen Corp., Rockville, MD) adjusted to contain 1.5 g/L sodium bicarbonate, 0.1 mM non-essential amino acids, and 1.0 mM sodium pyruvate, 90%; heat-inactivated fetal calf serum, 10%. The cells were incubated at 37°C, 8% CO2.

Twenty-four hours before transfection, HEK 293 cells were adhered to a 10 cm dish (Coming 430167, Coming Inc., Coming, NY) at 37°C, 8% CO2. The cells were co-transfected with human TLR7 or Empty Vector control pIRES (BD Biosciences Clontech, Palo Alto, CA) along with NFkB-luc reporter (Stratagene, La Jolla, CA) in a 10:1 ratio with Fugene 6 transfection reagent (Roche Diagnostics Corp., Indianapolis, IN) following the manufacturer's instructions. The plates were incubated for 24 hours following transfection and then selected in G-418 (400ug/mL) for 2 weeks. The G-418 resistant cells containing either the TLR7 or empty vector were expanded in HEK 293 media supplemented with G-418 for stimulation experiments.

TLR7 or empty vector cells were plated in white opaque 96 well plates (Costar 3917, Corning Inc., Coming, NY) at a concentration of 5 x$10^4$ cells per well in 100 μL of HEK 293 media and incubated at 37°C, 8% CO2 for 4 hours. The cells were stimulated with 1 μL of IRM compounds at 1mM in DMSO (final concentration of 10 μM) or 1 μL DMSO as a control. The plates were then incubated an additional 16 hours at 37°C, 5% CO2. The luciferase signal was read using the LucLite kit (Packard Instrument Co., Meriden, CT). The luminescence was measured on the Topcount NXT

(Packard Instrument Co., Meriden, CT).

**Table 6**

| | Fold Increase Over DMSO Control | |
|---|---|---|
| **Stimulus** | **HEK 293 Vector** | **HEK293 TLR7** |
| Imiquimod | 1.41 | 17.80 |
| IRM 9 | 0.81 | 4.67 |
| IRM 10 | 1.10 | 2.55 |
| IRM 11 | 1.35 | 1.06 |
| IRM 2 | 1.27 | 0.94 |
| IRM 1 | 0.86 | 3.75 |
| IRM 12 | 1.33 | 15.33 |
| IRM 4 | 1.00 | 4.06 |
| IRM 5 | 1.21 | 1.13 |
| IRM 6 | 0.95 | 1.25 |
| IRM 7 | 1.30 | 3.06 |
| IRM 8 | 0.91 | 4.59 |
| IRM 13 | 1.20 | 2.39 |
| IRM 14 | 1.31 | 1.37 |
| IRM15 | 1.04 | 1.88 |
| IRM 16 | 0.98 | 1.51 |
| IRM 17 | 0.99 | 2.79 |
| IRM 17 | 1.67 | 2.71 |
| DMSO | 1.00 | 1.00 |

## Claims

**1.** *N*-[4-(4-amino-2-ethyl-1*H*-imidazo[4,5-*c*]quinolin-1-yl)butyl]methanesulfonamide, or a pharmaceutically acceptable salt thereof.

**2.** A pharmaceutical composition comprising a therapeutically effective amount of a compound or salt of claim 1 in combination with a pharmaceutically acceptable carrier.

**3.** A compound or salt of claim 1 for use for inducing cytokine biosynthesis in an animal.

**4.** A compound or salt of claim 1 for use for treating a viral disease in an animal.

**5.** A compound of salt of claim 1 for use for treating a neoplastic disease in an animal.

## Patentansprüche

**1.** N-[4-(4-Amino-2-ethyl-1H-imidazo[4,5-c]chinolin-1-yl)butyl]methansulfonamid oder pharmazeutisch verträgliches Salz davon.

**2.** Pharmazeutische Zusammensetzung umfassend eine therapeutisch wirksame Menge einer Verbindung oder eines Salzes nach Anspruch 1 in Kombination mit einem pharmazeutisch verträglichen Träger.

**3.** Verbindung oder Salz nach Anspruch 1 zur Verwendung zum Induzieren der Cytokinbiosynthese bei einem Tier.

4. Verbindung oder Salz nach Anspruch 1 zur Verwendung zur Behandlung einer viralen Erkrankung bei einem Tier.

5. Verbindung oder Salz nach Anspruch 1 zur Verwendung zur Behandlung einer neoplastischen Erkrankung bei einem Tier.

**Revendications**

1. *N*-[4-(4-amino-2-éthyl-1*H*-imidazo[4,5-*c*]quinoléin-1-yl)butyl]méthanesulfonamide, ou un sel pharmaceutiquement acceptable de celui-ci.

2. Composition pharmaceutique comprenant une quantité thérapeutiquement efficace d'un composé ou d'un sel de la revendication 1, en combinaison avec un support pharmaceutiquement acceptable.

3. Composé ou sel de la revendication 1, destiné à être utilisé pour induire la biosynthèse de cytokines chez un animal.

4. Composé ou sel de la revendication 1, destiné à être utilisé pour traiter une maladie virale chez un animal.

5. Composé ou sel de la revendication 1, destiné à être utilisé pour traiter une maladie néoplasique chez un animal.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4689338 A
- US 4929624 A
- US 5266575 A
- US 5268376 A
- US 5352784 A
- US 5389640 A
- US 5482936 A
- US 5494916 A
- US 6110929 A
- US 6194425 A
- US 4985815 A
- US 5175296 A
- US 5367076 A
- US 5395937 A
- US 5693811 A
- US 5741908 A
- US 5238944 A
- US 5939090 A
- US 6245776 A
- US 6039969 A
- US 6083969 A
- US 6331539 A
- US 6376669 A
- WO 0076505 A
- WO 0076518 A
- WO 0246188 A
- WO 0246189 A
- WO 0246190 A
- WO 0246191 A
- WO 0246192 A
- WO 0246193 A
- WO 0246194 A
- WO 0076519 A
- US 637650 A
- US 6028076 A
- US 6329381 B
- US 6069149 A
- US 61994388 B
- US 6207646 B
- US 6239116 B
- US 6339068 B
- US 6406705 B
- WO 0075304 A
- US 6200592 A
- US 6083505 A
- US 6406705 A
- US 6194425 B
- EP 06010882 A
- US 60332412 B


### Non-patent literature cited in the description


- **TOMAI et al.** *Antiviral Research,* 1995, vol. 28 (3), 253-264
- **TOMAI et al.** *Antiviral Research,* 1995, vol. 28 (3), 253-64
- **MEGYERI et al.** *Molecular and Cellular Biology,* 1995, vol. 15 (4), 2207-18
- Current Protocols in Molecular Biology. John Wiley and Sons, Inc, 2001
- Current Protocols in Immunology. John Wiley and Sons, Inc, 2001